# EUROPEAN PATENT APPLICATION

(11) **EP 2 028 191 A1**
(43) Date of publication of application: **25.02.2009**
(21) Application number: 08075855.0
(22) Date of filing: 09.12.1997
(51) Int. Cl.: C07K 14/52, C07K 17/08, A61K 38/19

(54) **Peptides and compounds that bind to a receptor**

(30) Priority: 11.12.1996 US 764640
(62) Divisional of application: 97954363.4
(71) Applicant: Glaxo Group Limited, Greenford, Middlesex UB6 0NN (GB)
(72) Inventor: Dower, William J., Menlo Park, CA 94025 (US); Barrett, Ronald W., Saratoga, CA 95070 (US); Cwirla, Steven E., Menlo Park, CA 94025 (US); Gates, Christian M., Santa Cruz, Ca 95065 (US); Schatz, Peter J., Mountain View, CA 94040 (US); Balasubramanian, Palaniappan, Cupertino, CA 95014 (US); Wagstrom, Christopher R., Los Altos, CA 94024 (US); Hendren, Richard Wayne, Cary, NC 27511 (US); Deprince, Randolph B., Durham, NC 27705 (US); Podduturi, Surekha, San Jose, CA 95134 (US); Yin, Qun, San Jose, CA 95131 (US)
(74) Representative: Mercer, Christopher Paul

(57) **Abstract**

Described are peptides and compounds that bind to and activate the thrombopoietin receptor. Such peptides and compounds are useful in methids for treating haemoatological disorders and particularly, thrombocytopenia resulting from chemotheraoy, radiation therapy, or bone marrow transfusions as well as in diagnostic methods employing labelled peptides and compounds.

## Description

### BACKGROUND OF THE INVENTION

The present invention provides peptides and compounds that bind to and activate the thrombopoietin receptor (c-mpl or TPO-R) or otherwise act as a TPO agonist. The invention has application in the fields of biochemistry and medicinal chemistry and particularly provides TPO agonists for use in the treatment of human disease.

Peptides and compounds which bind to and activate the thrombopoietin receptor have been described in WO96/40750.

The slow recovery of platelet levels in patients suffering from thrombocytopenia is a serious problem, and has lent urgency to the search for a blood growth factor agonist able to accelerate platelet regeneration. The present invention provides such an agonist.

### SUMMARY OF THE INVENTION

The present invention provides compounds of formula (I) 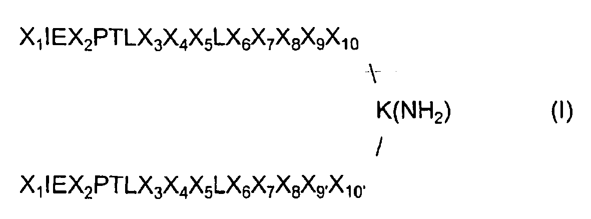 where:
X₁ is hydrogen or acyl;
X₂ is G or sarcosine (Sar);
X₃ is R, A, norleucine (Nle) or N-acetyllysine (Ac-Lys);
X₄ is Q or E;
X₅ is W, L-1-naphthylalanine (1-Nal) or F;
X₆ is A, 5-aminopentanoic acid (Ava) or 2-aminobutyric acid (Abu);
X₇ is A, diphenylalanine (Diphe) or X₇ is absent;
X₈ is R, p-aminophenylalanine (p-amino-Phe), N-acetyllysine (Ac-Lys) or X₈ is absent;
X₉ and X₉, are the same or different and are A, βA, n-methylalanine (n-Me-Ala), sarcosine (Sar), or X₉ or X₉. is absent;
X₁₀ and X₁₀, are the same or different and are βA, or X₁₀ or X₁₀, is absent;
and pharmaceutically acceptable derivatives thereof;
with the proviso that the compound 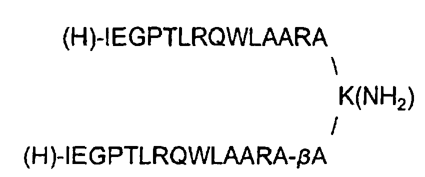 is excluded.

The invention also provides the compounds:

(H)-ADGPTLREWISF(Ava)ADGPTLREWISF(NH₂);

and 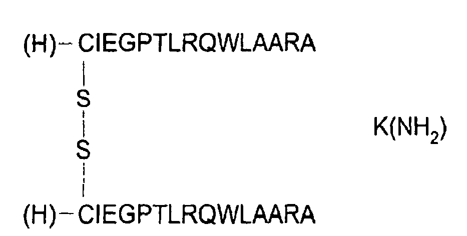 and pharmaceutically acceptable derivatives thereof.

In the above definition and hereinafter the symbols A, βA, C, D, E, F, G, I, K, L, P, Q, R, S, W, T are the standard one-letter codes for common amino acids, ie
- A: Alanine
- C: Cysteine
- D: Aspartic acid
- E: Glutamic acid
- F: Phenylalanine
- G: Glycine
- I: Isoleucine
- K: Lysine
- L: Leucine
- P: Proline
- Q: Glutamine
- R: Arginine
- S: Serine
- W: Tryptophan
- T: Threonine

For the avoidance of doubt, the abbreviation 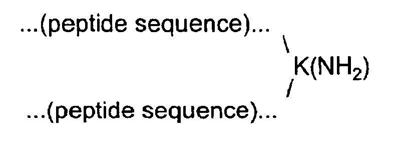 whenever used herein is meant to indicate the structure

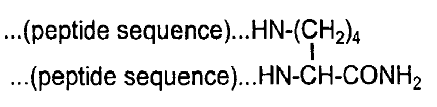

Suitable pharmaceutically acceptable derivatives of the compounds of formula (I) include pharmaceutically acceptable salts and acid addition salts, pharmaceutically acceptable esters, pharmaceutically acceptable amides, labelled compounds and compounds that are covalently attached to one or more of a variety of hydrophilic polymers as defined hereinafter.

Preferably the compounds of formula (I) are covalently attached to one or more, eg. one, of a variety of hydrophilic polymers. The hydrophilic polymer(s) may be attached, for example, to one or both of the peptide chains in the compounds of formula (I). If a hydrophilic polymer is attached to both peptide chains, then the hydrophilic polymers may be the same or different, preferably they will be the same. It will be appreciated by those skilled in the art that when the compounds of formula (I) are covalently attached to one or more of a variety of hydrophilic polymers at X₁. then X₁ is not acyl.

A preferred group of compounds of formula (I) is:- 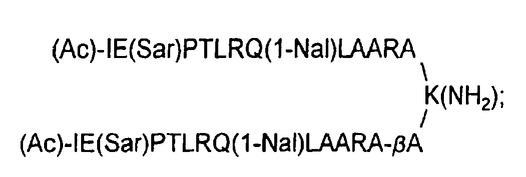 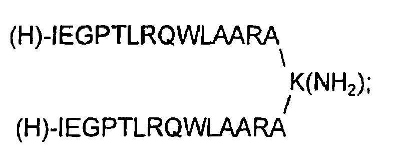 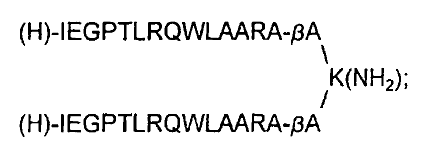 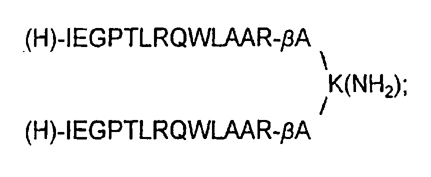 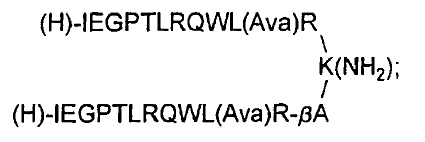 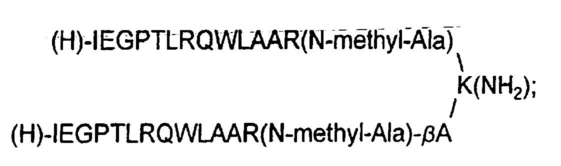 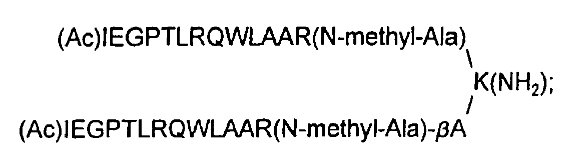 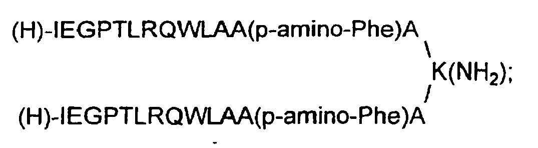 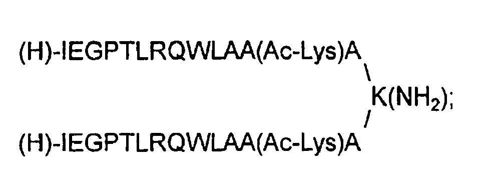 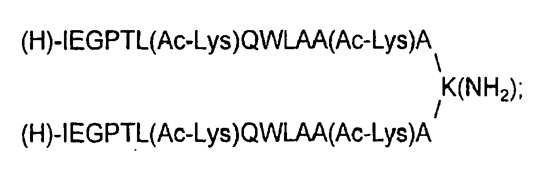 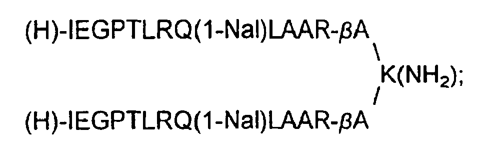 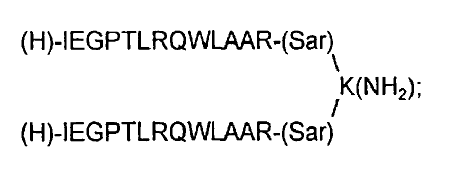 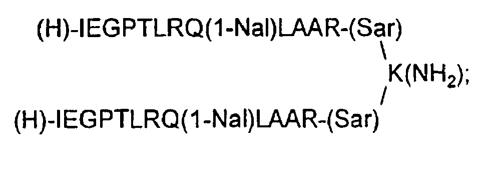 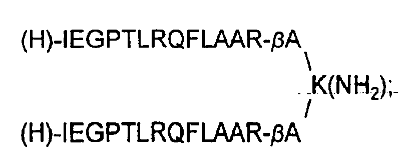 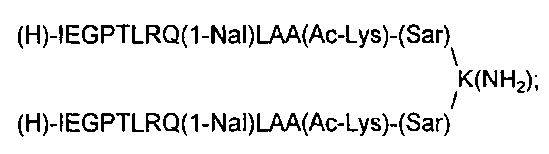 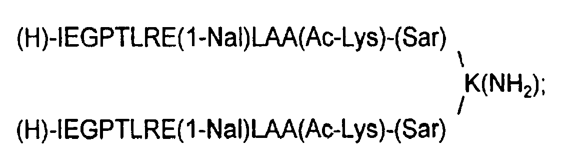 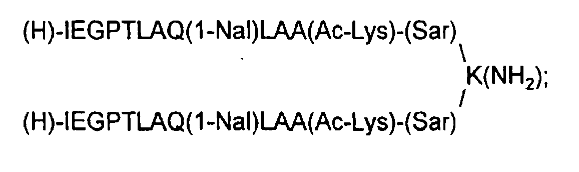 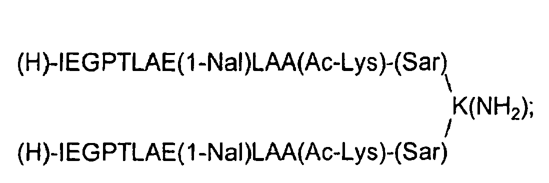 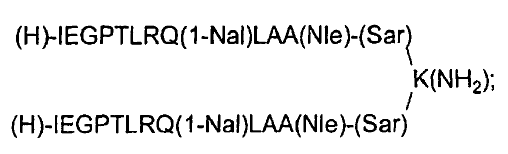 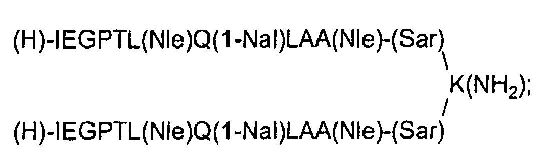 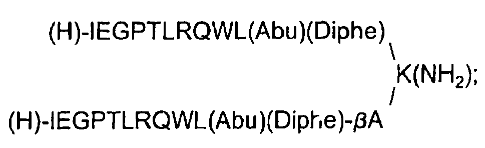 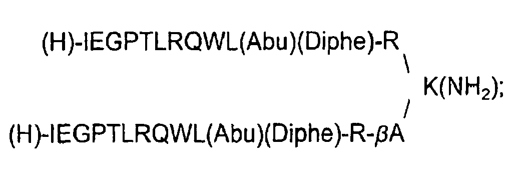 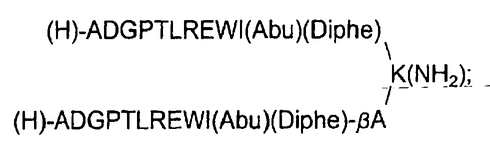 and pharmaceutically acceptable derivatives thereof, wherein the chiral amino acids are preferably in the L-form.

A further preferred group of compounds of formula (I) include: 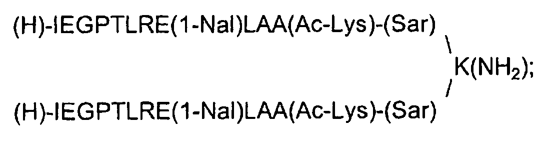 and especially 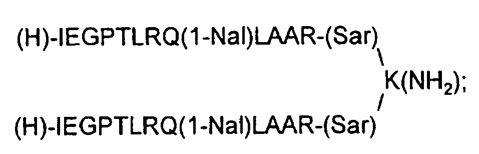 and pharmaceutically acceptable derivatives thereof, wherein the chiral amino acids are preferably in the L-form.

Reference hereinafter to a compound according to the invention includes both compounds of formula (I) and their pharmaceutically acceptable derivatives.

It will be appreciated by those skilled in the art that the compounds of formula (I) contain a number of chiral centres and therefore exist in the form of pairs of optical isomers (i.e. enantiomers) and mixtures thereof including racemic mixtures. All of the isomers of the compounds of formula (I) and mixtures thereof including racemic mixtures are included within the scope of the invention. Preferably chiral amino acids are in the L-form.

Compounds of formula (I) have strong binding properties to the TPO-R and can activate the TPO-R. Accordingly, such compounds are useful for therapeutic purposes in treating conditions which can be ameliorated by stimulation of TPO receptors, eg. on platelets, megakaryocytes and other stem cells, for example thrombocytopenia which may be as a result of, for example, chemotherapy (including induction, consolidation or maintenance chemotherapy), radiation therapy, autologous, allogeneic and syngeneic bone marrow transplantation, peripheral blood progenitor cell and cord blood progenitor cell transplantation, biological or other treatments for malignant and non-malignant disease (eg. interferon and other cytokines), bone marrow infiltration by metastatic tumours and leukaemia, bone marrow infiltration or encroachment in non-malignant diseases (eg. osteopetrosis, Gaucher's disease), aplastic anaemia, myelodysplastic syndrome, myelofibrosis, and related syndromes (both primary and secondary), immune and idiopathic disease, including autoimmune and alloimmune thrombocytopenias, and drug-induced immune thrombocytopenia, drug-induced thrombocytopenia (eg. due to anti-retroviral therapy in HIV-positive patients), chronic liver disease (eg. alcoholic and other forms of cirrhosis) and chronic renal failure, excessive platelet destruction, including thrombotic thrombocytopenic purpura, haemolytic uraemic syndrome and other microangiopathies, and hypersplenism, congenital syndromes where bleeding due to deficient or abnormal platelets occurs eg. Glanzmann's thrombasthenia, thrombocytopenia with absent radius (TAR) syndrome, gray platelet syndrome, liver transplantation, repair of aortic aneurysms, intra-aortic balloon counterpulsation, coronary artery bypass grafting and other surgical procedures requiring extracorporeal circulation and/or massive blood transfusion, or HIV-related thrombocytopenia; also granulocytopenia and anaemia which may be a result of, for example, chemotherapy, radiotherapy and other therapy for malignant and non-malignant diseases (as discussed above), bone marrow infiltration in malignant, pre-malignant and non-malignant diseases (as discussed above), immune and non-immune, idiopathic and drug-related anaemia and granulocytopenia; or the treatment of haematological malignancies *per se.* The compounds according to the invention may also be used for therapeutic, diagnostic and research purposes where stimulation of TPO receptors, eg. on platelets, megakaryocytes and other stem cells, is desirable for example in the mobilization of peripheral blood progenitor cells for autologous, allogeneic or syngeneic transplantation, increasing the yield from platelet and other blood donors, prolongation of platelet survival and quality ex vivo, production or expansion of platelets and/or progenitor cells *in vitro;* for diagnostic purposes in studying the mechanism of hematopoiesis and for the in *vitro* expansion of megakaroycytes and committed progenitor cells.

The compounds of the invention have an IC₅₀ of about 2 mM or less, as determined by the binding affinity assay set forth in Example 3 below wherein a lower IC₅₀ correlates to a stronger binding affinity to TPO-R. Preferably, for diagnostic purposes, the compounds according to the invention have an IC₅₀ of about 2 mM or less and, for pharmaceutical purposes, the compounds according to the invention have an IC₅₀ of about 100 µM or less.

When used for diagnostic purposes, the compounds according to the invention preferably are labelled with a detectable label and, accordingly, the compounds of the invention without such a label serve as intermediates in the preparation of labelled compounds.

If the compounds according to the invention are derivatized with a hydrophilic polymer as described herein, the molecular weights of such compounds can range anywhere from about 500 to about 120,000 daltons, more preferably from about 8,000 to about 80,000 daltons.

As discussed above, in a further preferred embodiment, compounds of formula (I) are covalently attached to one or more of a variety of hydrophilic polymers. Generally, such hydrophilic polymers have an average molecular weight ranging from about 500 to about 100,000 daltons, eg from about 500 to about 40,000 daltons, preferably from about 2,000 to about 40,000 daltons, more preferably from about 5,000 to about 40,000 and, even more preferably, from about 5,000 to about 20,000 daltons, eg about 18,800 to about 22,000 daltons. In preferred embodiments, such hydrophilic polymers have average molecular weights of about 5,000 daltons, 10,000 daltons and 20,000 daltons. The hydrophylic polymers may be branched or non-branched.

Suitable hydrophilic polymers include, but are not limited to, polyalkylethers as exemplified by polyethylene glycol and polypropylene glycol, polylactic acid, polyglycolic acid, polyoxyalkenes, polyvinylalcohol, polyvinylpyrrolidone, cellulose and cellulose derivatives, dextran and dextran derivatives, and copolymers thereof *etc*. Preferred hydrophilic polymers include polyalkylethers as exemplified by polyethylene glycol and polypropylene glycol, polylactic acid, polyglycolic acid, and copolymers thereof. It has surprisingly been discovered that when the peptide compounds are derivatized with such polymers, their solubility and circulation half-lives are increased with little, if any, diminishment in their binding activity and their immunogenicity is masked. In a preferred embodiment, each of the dimeric subunits (peptide chains) of the peptide compounds of formula (I) is covalently attached to a hydrophilic polymer. In a further preferred embodiment, the compounds of this invention are PEGylated. *i*.*e*., covalently attached to polyethylene glycol (PEG).

PEG is a linear, water-soluble polymer of ethylene oxide repeating units with two terminal hydroxyl groups. PEGs are classified by their molecular weights which typically range from about 500 daltons to about 40,000 daltons for example, 5,000, 10,000, 20,000, 30,000 or 40,000 daltons (5K, 10K, 20K, 30K or 40K). In a presently preferred embodiment, the PEGs employed have molecular weights ranging from 5,000 daltons to about 20,000 daltons. PEGs coupled to the peptide compounds of the present invention can be either branched or unbranched. (See, *e.g*., Monfardini, C., *et al., Bioconjugate Chem.,* ***6*:62-69** (1995)). Such PEGs include, but are not limited to, monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), and monomethoxypolyethylene glycol-imidazolyl-carbonyl (MePEG-IM).

The compounds according to the invention may be mono- or di-pegylated, for example monopegylation may be preferred where a PEG of high molecular weight is employed.

Examples of PEG chains that may be attached to the compounds of formula (I) include:
CH₃O-(CH₂CH₂O)ₙ-CH₂CH₂-CH₂-NH- Aldehyde-linked (ALDH) PEG where n is for example about 450
CH₃O-(CH₂CH₂O)ₙ-CH₂CH₂-C(O)-NH- Ester-linked (SPA) PEG where n is for example about 112 - 900, eg about 112, 225, 450 (eg 425 - 500), 675 or 900 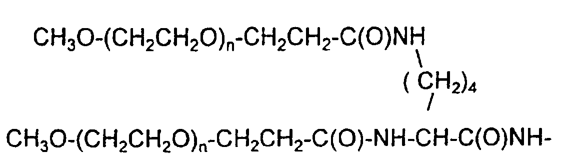 Branched PEG where n is for example about 112 - 450, eg about 112, 225 or 450
-CH₃O-(CH₂CH₂O)ₙ-CH₂CH₂-OC(O)CH₂CH₂-C(O)NH- SS PEG where n is for example about 112 - 450, eg about 112, 225 or 450

Examples of preferred PEGylated, compounds of this invention include, but are not limited to, the following: 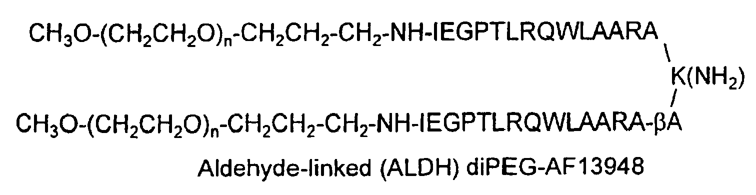 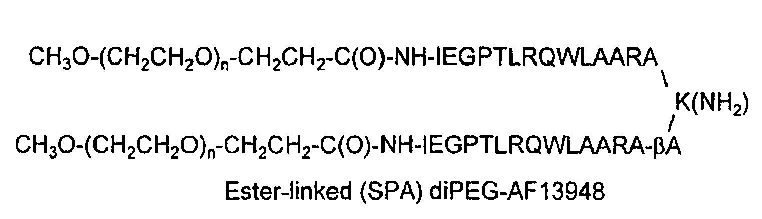 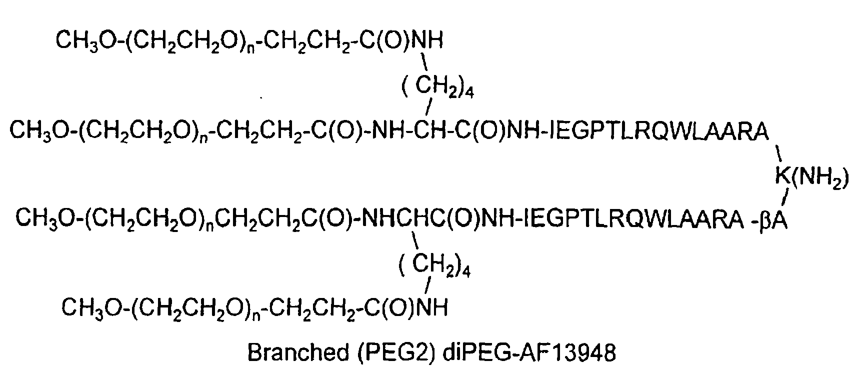 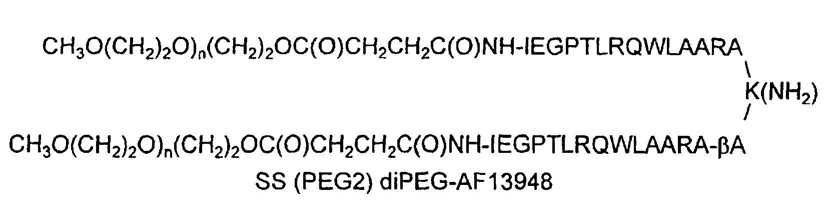 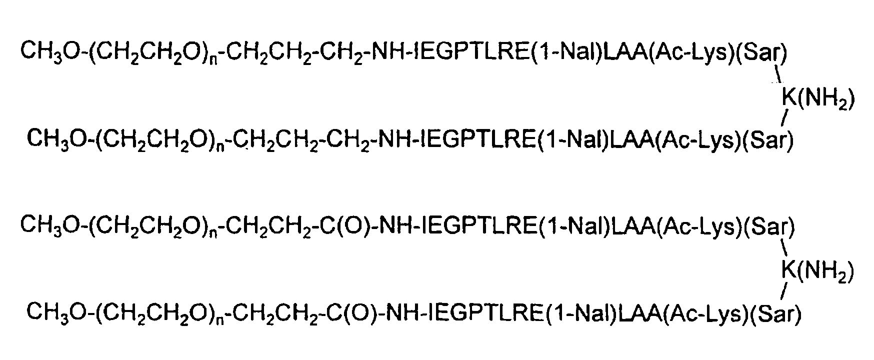 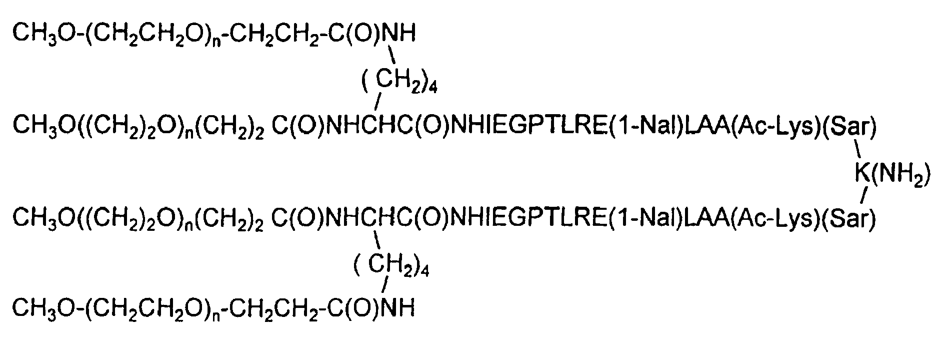 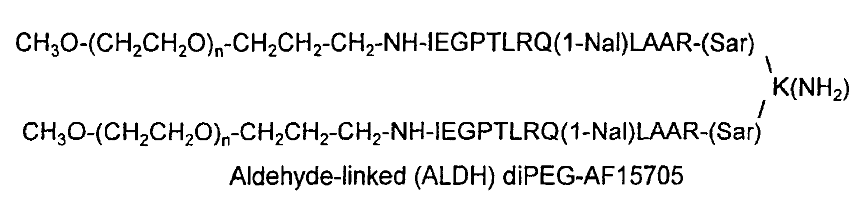 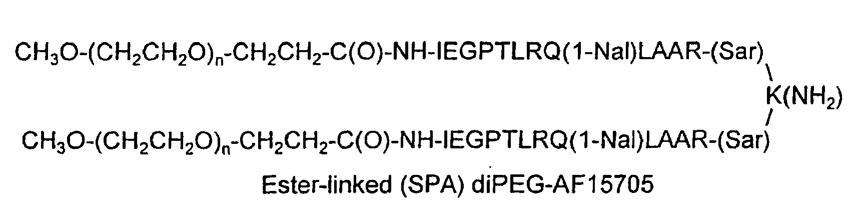 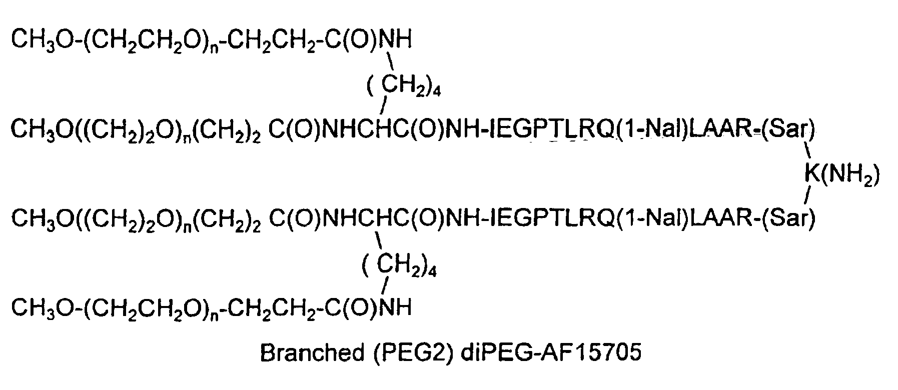 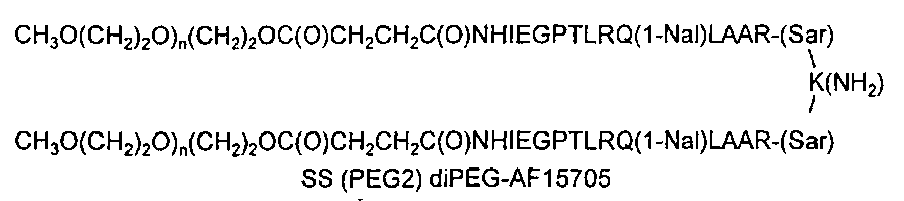 and pharmaceutically acceptable derivatives thereof, wherein the chiral amino acids are preferably in the L-form and wherein "n" is an integer having a value ranging from about 5 to about 1000, e.g. 10 to about 1000, more preferably from about 100 to about 900 and, even more preferably, from about 110 to about 900, eg about 112, 225, 450 (eg, 425-500), 675 or 900. For example for aldehyde-linked (ALDH) PEGs, n is for example about 450, eg about 348 - 452; for ester-linked (SPA) PEGs, n is for example about 112 - 900, eg about 112, 225, 450 (eg, 425-500), 675 or 900; for branched PEGs, n is for example about 112 - 450, eg about 112, 225 or 450; and for SS PEGs, n is for example about 112 - 450, eg about 112, 225 or 450.

A particularly preferred compound according to the invention is: 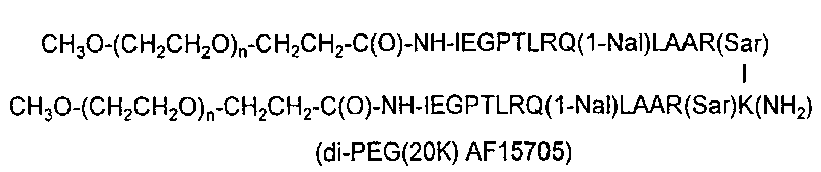 where n is about 450, eg about 425 - 500, and pharmaceutically acceptable derivatives thereof, wherein the chiral amino acids are preferably in the L-form.

As discussed hereinabove the compounds according to the invention are useful for the prevention and treatment of diseases mediated by TPO, for example haematological disorders including thrombocytopenia, granulocytopenia and anaemia, and the treatment of haematological malignancies. Thus, the present invention also provides a method for treating a patient suffering from a disorder that is susceptible to treatment with a thrombopoietin agonist comprising administering to the patient a therapeutically effective dose or amount of a compound of the present invention.

The invention further provides a compound according to the invention for use in therapy, in particular in human medicine.

There is also provided as a further aspect of the invention the use of a compound according to the invention in the preparation of a medicament for use in the treatment of conditions mediated by thrombopoietin agonists.

It will be appreciated that reference to treatment is intended to include prophylaxis as well as the alleviation of established symptoms.

The invention also provides for pharmaceutical compositions comprising one or more of the compounds described herein and a physiologically acceptable carrier. These pharmaceutical compositions can be in a variety of forms including oral dosage forms, as well as inhalable powders and solutions and injectable and infusible solutions.

### BRIEF DESCRIPTION OF THE FIGURES

Figure 1 illustrates the stability of AF13948 and GW350781 (di-PEG(5K)-AF13948) in human serum and demonstrates that the PEGylated compound has increased stability, *i.e.,* increased half-life, over the non-PEGylated compound.
Figure 2 illustrates the pharmacokinetic profiles of a peptide compound of the present invention variously derivatized with PEG. In this experiment, the peptide AF15705 was derivatized with branched PEG (PEG2), with an ester linked PEG (SPA) and with an aldehyde linked PEG (ALDH). The results obtained indicate that all three of the peptide compounds variously derivatized with PEG have favorable pharmacokinetic profiles.
Figures 3-4 illustrate the effects of the PEGylated peptide compounds of the present invention on carboplatin (CBP)-induced thrombocytopenia in mice. Figure 3 demonstrates that GW350781 (di-PEG(5K)-AF13948) can ameliorate thrombocytopenia in a mouse model. Figure 4 demonstrates that GW350805 (di-PEG(20K)-AF13948) can also ameliorate carboplatin-induced thrombocytopenia and that it is even more potent than GW350781.
Figures 5-6 illustrate the effects of GW350781 and GW350805 on thrombocytosis in normal mice. The results obtained indicate that the PEGylated peptide compounds of the invention have a favorable effect on thrombocytosis, with the 20K-diPEGylated peptide being about 100-fold more potent than the 5K-diPEGylated peptide.
Figures 7-8 illustrate the effects of varying doses of GW350781 and GW350805 on platelet levels in normal mice. Such data demonstrate that the PEGylated peptide compounds of the invention can increase platelet levels in normal mice.
Figure 9 illustrates the effect of single-dose vs. multiple-dose of GW350805 on platelet levels in normal mice.
Figure 10 illustrates a general synthetic scheme for making dimer peptides with β-Ala.
Figure 11 illustrates a general synthetic scheme for making dimer peptides without β-Ala.

### DESCRIPTION OF SPECIFIC EMBODIMENTS

The following definitions are set forth to illustrate and define the meaning and scope of the various terms used to describe the invention herein.

"Agonist" refers to a biologically active ligand which binds to its complementary biologically active receptor and activates the latter either to cause a biological response in the receptor or to enhance preexisting biological activity of the receptor.

"Pharmaceutically acceptable salts" refer to the non-toxic alkali metal, alkaline earth metal, and ammonium salts commonly used in the pharmaceutical industry including the sodium, potassium, lithium, calcium, magnesium, barium, ammonium, and protamine zinc salts, which are prepared by methods well known in the art. The term also includes non-toxic acid addition salts, which are generally prepared by reacting the compounds of this invention with a suitable organic or inorganic acid. Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate, and the like.

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. For a description of pharmaceutically acceptable acid addition salts as prodrugs, see Bundgaard, H., supra.

"Pharmaceutically acceptable ester" refers to those esters which retain, upon hydrolysis of the ester bond, the biological effectiveness and properties of the carboxylic acid or alcohol and are not biologically or otherwise undesirable. For a description of pharmaceutically acceptable esters as prodrugs, see Bundgaard, H., ed., Design of Prodrugs, Elsevier Science Publishers, Amsterdam (1985). These esters are typically formed from the corresponding carboxylic acid and an alcohol. Generally, ester formation can be accomplished via conventional synthetic techniques. (See, *e*.*g*., March, Advanced Organic Chemistry, 4th Ed., John Wiley & Sons, New York (1992), 393-396 and references cited therein, and Mark, et al., Encyclopedia of Chemical Technology, John Wiley & Sons, New York (1980).) The alcohol component of the ester will generally comprise (i) a C₂-C₁₂ aliphatic alcohol that can or can not contain one or more double bonds and can or can not contain branched carbons or (ii) a C₇-C₁₂ aromatic or heteroaromatic alcohols. This invention also contemplates the use of those compositions which are both esters as described herein and at the same time are the pharmaceutically acceptable acid addition salts thereof.

"Pharmaceutically acceptable amide" refers to those amides which retain, upon hydrolysis of the amide bond, the biological effectiveness and properties of the carboxylic acid or amine and are not biologically or otherwise undesirable. For a description of pharmaceutically acceptable amides as prodrugs, see Bundgaard, H., ed., Design of Prodrugs, Elsevier Science Publishers, Amsterdam (1985). These amides are typically formed from the corresponding carboxylic acid and an amine. Generally, amide formation can be accomplished via conventional synthetic techniques. (See, e.g., March, Advanced Organic Chemistry, 4th Ed., John Wiley & Sons, New York (1992), p. 393 and Mark, et al. Encyclopedia of Chemical Technology, John Wiley & Sons, New York (1980).) This invention also contemplates the use of those compositions which are both amides as described herein and at the same time are the pharmaceutically acceptable acid addition salts thereof.

"Pharmaceutically or therapeutically acceptable carrier" refers to a carrier medium which does not interfere with the effectiveness of the biological activity of the active ingredients and which is not toxic to the host or patient.

"Stereoisomer" refers to a chemical compound having the same molecular weight, chemical composition, and constitution as another, but with the atoms grouped differently. That is, certain identical chemical moieties are at different orientations in space and, therefore, when pure, has the ability to rotate the plane of polarized light. However, some pure stereoisomers may have an optical rotation that is so slight that it is undetectable with present instrumentation. The compounds of the instant invention may have one or more asymmetrical carbon atoms and therefore include various stereoisomers. All stereoisomers are included within the scope of the invention.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve a decrease in the immunological and/or inflammatory responses to infection or tissue injury.

The following abbreviations are used:
OtBu and tBu are both tert-butyloxy (OtBu is conventionally used with 3 letter amino acid abbreviations and tBu conventionally used with 1 letter amino acid abbreviations), Bzl is benzyl, Ac is acetyl, Me is methyl, Abu is 2-aminobutyric acid, Thi is thienylalanine, Ac-Lys is N-acetyllysine, p-amino-Phe is p-aminophenylalanine, N-methyl-Ala is N-methylalanine, Diphe is diphenylalanine, Sar is sarcosine, Ava is 5-aminopentanoic acid, Nle is norleucine, trt is trityl.

"Detectable label" refers to materials, which when covalently attached to the peptides and peptide mimetics of this invention, permit detection of the peptide and peptide mimetics *in vivo* in the patient to whom the peptide or peptide mimetic has been administered. Suitable detectable labels are well known in the art and include, by way of example, radioisotopes, fluorescent labels (e.g., fluorescein), and the like. The particular detectable label employed is not critical and is selected relative to the amount of label to be employed as well as the toxicity of the label at the amount of label employed. Selection of the label relative to such factors is well within the skill of the art.

Covalent attachment of the detectable label to the peptide or peptide mimetic is accomplished by conventional methods well known in the art. For example, when the ¹²⁵I radioisotope is employed as the detectable label, covalent attachment of ¹²⁵I to the peptide or the peptide mimetic can be achieved by incorporating the amino acid tyrosine into the peptide or peptide mimetic and then iodimating the peptide (see, e.g., Weaner, et al., Synthesis and Applications of Isotopically Labelled Compounds, pp. 137-140 (1994)). If tyrosine is not present in the peptide or peptide mimetic, incorporation of tyrosine to the N or C terminus of the peptide or peptide mimetic can be achieved by well known chemistry. Likewise, ³²P can be incorporated onto the peptide or peptide mimetic as a phosphate moiety through, for example, a hydroxyl group on the peptide or peptide mimetic using conventional chemistry.

The present invention provides compounds that bind to and activate the TPO-R or otherwise behave as a TPO agonist. These compounds include "lead" peptide compounds and "derivative" compounds constructed so as to have the same or similar molecular structure or shape as the lead compounds but that differ from the lead compounds either with respect to susceptibility to hydrolysis or proteolysis and/or with respect to other biological properties, such as increased affinity for the receptor. The present invention also provides compositions comprising an effective amount of a TPO agonist, and more particularly a compound, that is useful for treating hematological disorders, and particularly, thrombocytopenia associated with chemotherapy, radiation therapy, or bone marrow transfusions.

A variety of methods can be used to evaluate IC₅₀ values. For example, an equilibrium binding ELISA assay, using either MBP-TPO or lacl-peptide tracer, was used to determine whether the peptides inhibit the binding of TPO to the extracellular domain of the TPO receptor. The IC₅₀ value can be determined using the free peptide, which optionally can be C-terminally amidated, or can be prepared as an ester or other carboxy amide.

IC₅₀ and EC₅₀ values are indicated symbolically herein by the symbols "-", "+", and "++". For examples, those peptides which showed IC₅₀ values in excess of 200 µM are indicated with a "-". Those peptides which gave IC₅₀ values of less than or equal to 200 µM are given a "+", while those which gave IC₅₀ values of 500 nm or less are indicated with a "++". Those peptides which gave IC₅₀ values at or near the cutoff point for a particular symbol are indicated with a hybrid designator, *e.g.,* "+/-". Those peptides for which IC₅₀ values were not determined are listed as "N.D."

The peptides and peptide mimetics of the present invention were also evaluated in a thrombopoietin dependent cell proliferation assay, as described in greater detail in Example 2 below. Cell proliferation is measured by techniques known in the art, such as an MTT assay which correlates with ³H-thymidine incorporation as an indication of cell proliferation (see Mossmann, J. Immunol. Methods, 65:55 (1983)).

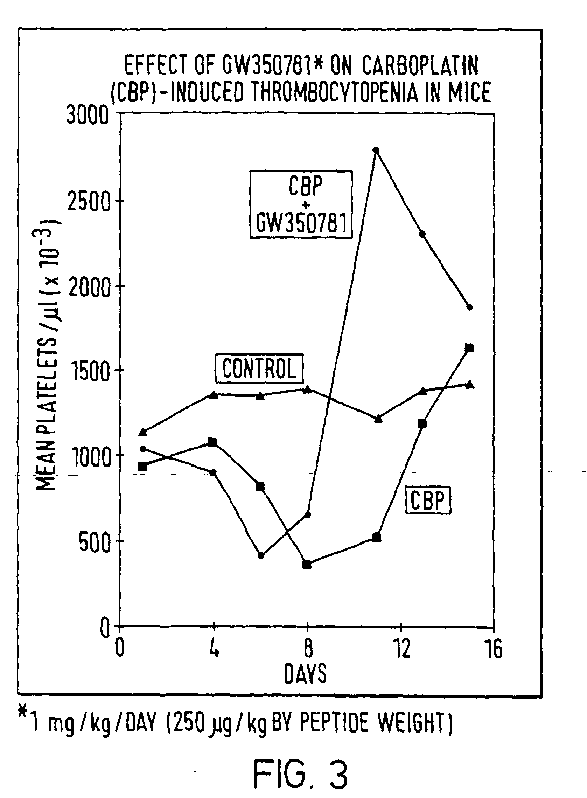

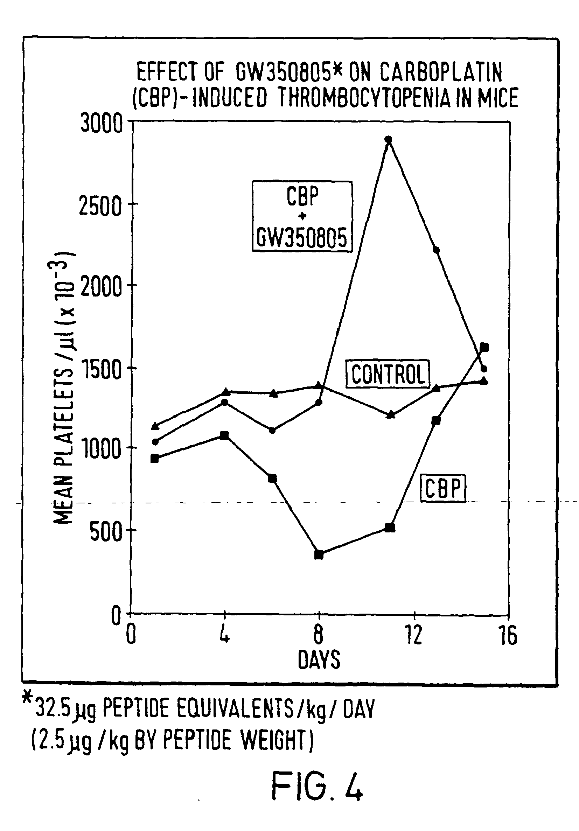

Figures 3-4 show the results of a further assay evaluating activity of the peptides and peptide mimetics of the invention. In this assay mice are made thrombocytopenic with carboplatin. Balb/C mice are treated with carboplatin (125 mg/kg intraperitoneally) on Day 0. These results show the peptides of the invention can ameliorate thrombocytopenia in a mouse model.

The specificity of the binding and activity of the peptides of the invention was also examined by studying the cross reactivity of the peptides for the erythropoietin receptor (EPO-R) according to the method described in WO96/40750.

### PREPARATION OF PEPTIDES AND PEPTIDE MIMETICS SOLID PHASE SYNTHESIS

The peptides of the invention can be prepared by classical methods known in the art, for example, by using standard solid phase techniques. The standard methods include exclusive solid phase synthesis, partial solid phase synthesis methods, fragment condensation, classical solution synthesis, and even by recombinant DNA technology. *See*, *e.g.,* Merrifield, J. Am. Chem. Soc., 85:2149 (1963), incorporated herein by reference. On solid phase, the synthesis is typically commenced from the C-terminal end of the peptide using an alpha-amino protected resin. A suitable starting material can be prepared, for instance, by attaching the required alpha-amino acid to a chloromethylated resin, a hydroxymethyl resin, or a benzhydrylamine resin. One such chloromethylated resin is sold under the tradename BIO-BEADS SX-1 by Bio Rad Laboratories, Richmond, CA, and the preparation of the hydroxymethyl resin is described by Bodonszky, et al., Chem. Ind. (London), 38:1597 (1966). The benzhydrylamine (BHA) resin has been described by Pietta and Marshall, Chem. Commn., 650 (1970) and is commercially available from Beckman Instruments, Inc., Palo Alto, CA, in the hydrochloride form.

Thus, the compounds of the invention can be prepared by coupling an alpha-amino protected amino acid to the chloromethylated resin with the aid of, for example, cesium bicarbonate catalyst, according to the method described by Gisin, Helv. Chim. Acta., 56:1467 (1973). After the initial coupling, the alpha-amino protecting group is removed by a choice of reagents including trifluoroacetic acid (TFA) or hydrochloric acid (HCl) solutions in organic solvents at room temperature.

The alpha-amino protecting groups are those known to be useful in the art of stepwise synthesis of peptides. Included are acyl type protecting groups (*e*.*g*., formyl, trifluoroacetyl, acetyl), aromatic urethane type protecting groups (*e*.*g*. benzyloxycarboyl (Cbz) and substituted Cbz), aliphatic urethane protecting groups (*e*.*g*., t-butyloxycarbonyl (Boc), isopropyloxycarbonyl, cyclohexyloxycarbonyl) and alkyl type protecting groups (*e*.*g*., benzyl, triphenylmethyl). Boc and Fmoc are preferred protecting groups. The side chain protecting group remains intact during coupling and is not split off during the deprotection of the amino-terminus protecting group or during coupling. The side chain protecting group must be removable upon the completion of the synthesis of the final peptide and under reaction conditions that will not alter the target peptide.

The side chain protecting groups for Thr (T) and Ser include acetyl, benzoyl, trityl, tetrahydropyranyl, benzyl, 2,6-dichlorobenzyl, and Cbz. The side chain protecting groups for Arg (R) include nitro, Tosyl (Tos), Cbz, adamantyloxycarbonyl mesitoylsulfonyl (Mts), Pmc or Boc. The side chain protecting groups for Lys (K) include Cbz, 2-chlorobenzyloxycarbonyl (2-Cl-Cbz), 2-bromobenzyloxycarbonyl (2-BrCbz), Tos, or Boc.

After removal of the alpha-amino protecting group, the remaining protected amino acids are coupled stepwise in the desired order. An excess of each protected amino acid is generally used with an appropriate carboxyl group activator such as dicyclohexylcarbodiimide (DCC) in solution, for example, in methylene chloride (CH₂Cl₂), dimethyl formamide (DMF) mixtures.

After the desired amino acid sequence has been completed, the desired peptide is decoupled from the resin support by treatment with a reagent such as trifluoroacetic acid or hydrogen fluoride (HF), which not only cleaves the peptide from the resin, but also cleaves all remaining side chain protecting groups. When the chloromethylated resin is used, hydrogen fluoride treatment results in the formation of the free peptide acids. When the benzhydrylamine resin is used, hydrogen fluoride treatment results directly in the free peptide amide. Alternatively, when the chloromethylated resin is employed, the side chain protected peptide can be decoupled by treatment of the peptide resin with ammonia to give the desired side chain protected amide or with an alkylamine to give a side chain protected alkylamide or dialkylamide. Side chain protection is then removed in the usual fashion by treatment with hydrogen fluoride to give the free amides, alkylamides, or dialkylamides.

These solid phase peptide synthesis procedures are well known in the art and further described by John Morrow Stewart and Janis Dillaha Young, *Solid Phase Peptide Syntheses* (2nd Ed., Pierce Chemical Company, 1984).

The compounds of the invention may also be prepared using the "encoded synthetic library" or "very large scale immobilized polymer synthesis" system described in U.S. Patent Application Serial Nos. 07/492,462, filed March 7, 1990; 07/624,120, filed December 6, 1990; and 07/805,727, filed December 6, 1991.

### B. SYNTHETIC AMINO ACIDS

These procedures can also be used to synthesize peptides in which amino acids other than the 20 naturally occurring, genetically encoded amino acids are substituted at one, two, or more positions of any of the compounds of the invention. For instance, naphthylalanine can be substituted for tryptophan, facilitating synthesis. Other synthetic amino acids that can be substituted into the peptides of the present invention include p amino acids, D amino acids and non-naturally occurring synthetic amino acids (see, *e.g.,* Roberts, et al., Unusual Amino/Acids in Peptide Synthesis, 5(6):341-449 (1983)).

### N-TERMINAL MODIFICATIONS

The peptides typically are synthesized as the free acid but, as noted above, could be readily prepared as the amide or ester. One can also modify the amino and/or carboxy terminus of the peptide compounds of the invention to produce other compounds of the invention. Amino terminus modifications include methylation (*i.e.,* -NHCH₃ or -NH(CH₃)₂), acetylation, adding a benzyloxycarbonyl group, or blocking the amino terminus with any blocking group containing a carboxylate functionality defined by RCOO-, where R is selected from the group consisting of naphthyl, acridinyl, steroidyl, and similar groups. Carboxy terminus modifications include replacing the free acid with a carboxamide group or forming a cyclic lactam at the carboxy terminus to introduce structural constraints.

Amino terminus modifications are as recited above and include alkylating, acetylating, adding a carbobenzoyl group, forming a succinimide group, etc. (See, *e*.*g*., Murray, et al., Burger's Medicinal Chemistry and Drug Discovery, 5th ed., Vol. 1, Manfred E. Wolf, ed., John Wiley and Sons, Inc. (1995).) Specifically, the N-terminal amino group can then be reacted as follows:
(a) to form an amide group of the formula RC(O)NH- where R is as defined above by reaction with an acid halide [*e*.*g*., RC(O)Cl] or symmetric anhydride. Typically, the reaction can be conducted by contacting about equimolar or excess amounts (*e*.*g*., about 5 equivalents) of an acid halide to the peptide in an inert diluent (*e*.*g*., dichloromethane) preferably containing an excess (*e*.*g*., about 10 equivalents) of a tertiary amine, such as diisopropylethylamine, to scavenge the acid generated during reaction. Reaction conditions are otherwise conventional (e.g., room temperature for 30 minutes). Alkylation of the terminal amino to provide for a lower alkyl N-substitution followed by reaction with an acid halide as described above will provide for N-alkyl amide group of the formula RC(O)NR-;
(b) to form a succinimide group by reaction with succinic anhydride. As before, an approximately equimolar amount or an excess of succinic anhydride (*e.g*., about 5 equivalents) can be employed and the amino group is converted to the succinimide by methods well known in the art including the use of an excess (*e*.*g*., ten equivalents) of a tertiary amine such as diisopropylethylamine in a suitable inert solvent (*e*.*g*., dichloromethane). See, for example, Wollenberg, et al., U.S. Patent No. 4,612,132 which is incorporated herein by reference in its entirety. It is understood that the succinic group can be substituted with, for example, C₂-C₆ alkyl or -SR substituents which are prepared in a conventional manner to provide for substituted succinimide at the N-terminus of the peptide. Such alkyl substituents are prepared by reaction of a lower olefin (C₂-C₆) with maleic anhydride in the manner described by Wollenberg, *et al., supra* and -SR substituents are prepared by reaction of RSH with maleic anhydride where R is as defined above;
(c) to form a benzyloxycarbonyl-NH- or a substituted benzyloxycarbonyl-NH-group by reaction with approximately an equivalent amount or an excess of CBZ-Cl (i.e., benzyloxycarbonyl chloride) or a substituted CBZ-Cl in a suitable inert diluent (*e*.*g*., dichloromethane) preferably containing a tertiary amine to scavenge the acid generated during the reaction;
(d) to form a sulfonamide group by reaction with an equivalent amount or an excess (*e*.*g*., 5 equivalents) of R-S(O)₂Cl in a suitable inert diluent (dichloromethane) to convert the terminal amine into a sulfonamide where R is as defined above. Preferably, the inert diluent contains excess tertiary amine (e*.g*., ten equivalents) such as diisopropylethylamine, to scavenge the acid generated during reaction. Reaction conditions are otherwise conventional (*e*.*g*., room temperature for 30 minutes);
(e) to form a carbamate group by reaction with an equivalent amount or an excess (*e*.*g*., 5 equivalents) of R-OC(O)Cl or R-OC(O)OC₆H₄-p-NO₂ in a suitable inert diluent (*e*.*g*., dichloromethane) to convert the terminal amine into a carbamate where R is as defined above. Preferably, the inert diluent contains an excess (*e*.*g*., about 10 equivalents) of a tertiary amine, such as diisopropylethylamine, to scavenge any acid generated during reaction. Reaction conditions are otherwise conventional (*e*.*g*., room temperature for 30 minutes); and
(f) to form a urea group by reaction with an equivalent amount or an excess (*e*.*g*., 5 equivalents) of R-N=C=O in a suitable inert diluent (*e*.*g*., dichloromethane) to convert the terminal amine into a urea (*i.e.,* RNHC(O)NH-) group where R is as defined above. Preferably, the inert diluent contains an excess (*e*.*g*., about 10 equivalents) of a tertiary amine, such as diisopropylethylamine. Reaction conditions are otherwise conventional (*e*.*g*., room temperature for about 30 minutes).

In another alternative embodiment, the C-terminal carboxyl group or a C-terminal ester can be induced to cyclize by internal displacement of the -OH or the ester (-OR) of the carboxyl group or ester respectively with the N-terminal amino group to form a cyclic peptide. For example, after synthesis and cleavage to give the peptide acid, the free acid is converted to an activated ester by an appropriate carboxyl group activator such as dicyclohexylcarbodiimide (DCC) in solution, for example, in methylene chloride (CH₂Cl₂), dimethyl formamide (DMF) mixtures. The cyclic peptide is then formed by internal displacement of the activated ester with the N-terminal amine. Internal cyclization as opposed to polymerization can be enhanced by use of very dilute solutions. Such methods are well known in the art.

One can also cyclize the peptides of the invention, or incorporate a desamino or descarboxy residue at the terminii of the peptide, so that there is no terminal amino or carboxyl group, to decrease susceptibility to proteases or to restrict the conformation of the peptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

In addition to the foregoing N-terminal and C-terminal modifications, the compounds of the invention can advantageously be modified with or covalently coupled to one or more of a variety of hydrophilic polymers as defined above.

The peptide compounds of the invention can be derivatized with or coupled to such polymers using any of the methods set forth in Zallipsky, S., Bioconjugate Chem., 6:150-165 (1995); Monfardini, C, et al., Bioconjugate Chem., 6:62-69 (1995); U.S. Patent No. 4,640,835; U.S. Patent No. 4,496,689; U.S. Patent No. 4,301,144; U.S. Patent No. 4,670,417; U.S. Patent No. 4,791,192; U.S. Patent No. 4,179,337 or WO 95/34326, all of which are incorporated by reference in their entirety herein. PEGs are commercially available from Shearwater Polymers, Inc. (Huntsville, Alabama), Sigma Chemical Co. and other companies.

In a presently preferred embodiment, the peptide compounds of the present invention are derivatized with polyethylene glycol (PEG).

Briefly, in one exemplar embodiment, the hydrophilic polymer which is employed, *e*.*g*., PEG, is preferably capped at one end by an unreactive group such as a methoxy or ethoxy group. Thereafter, the polymer is activated at the other end by reaction with a suitable activating agent, such as cyanuric halides (*e*.*g*., cyanuric chloride, bromide or fluoride), diimidazole, an anhydride reagent (*e*.*g*., a dihalosuccinic anhydride, such as dibromosuccinic anhydride), acyl azide, p-diazoiumbenzyl ether, 3-(p-diazoniumphenoxy)-2-hydroxypropylether) and the like. The activated polymer is then reacted with a peptide compound of the present invention to produce a peptide compound derivatized with a polymer. If one of the peptide arms of the compounds of the invention is protected, eg by an acyl group, a monopegylated compound will be formed. Alternatively, a functional group in the peptide compounds of the invention can be activated for reaction with the polymer, or the two groups can be joined in a concerted coupling reaction using known coupling methods. Schemes 1-4 illustrate exemplar reactions schemes for derivatizing the peptide compounds of the invention with, for example, polyethylene glycol (PEG). It will be readily appreciated that the peptide compounds of the invention can be derivatized with PEG using a myriad of other reaction schemes known to and used by those of skill in the art.

In addition to derivatizing the peptide compounds of this invention with a hydrophilic polymer (*e*.*g*., PEG), it has now been discovered that other small peptides, *e.g*., other peptides or ligands that bind to a receptor, can also be derivatized with such hydrophilic polymers with little, if any, loss in biological activity (*e*.*g*., binding activity, agonist activity, antagonist activity, *etc*.). It has been found that when these small peptides are derivatized with a hydrophlilic polymer, their solubility and circulation half-lives are increased and their immunogenicity is decreased. Again, quite surprisingly, the foregoing can be accomplished with little, if any, loss in biological activity. In fat, in preferred embodiments, the derivatized peptides have an activity that is 0.1 to 0.01-fold that of the unmodified peptides. In more preferred embodiments, the derivatized peptides have an activity that is 0.1 to 1-fold that of the unmodified peptides. In even more preferred embodiments, the derivatized peptides have an activity that is greater than the unmodified peptides.

Peptides suitable for use in this embodiment generally include those peptides, *i*.*e*., ligands, that bind to a receptor, such as the TPO, EPO, IL-1, G-CSF and IL-5 receptors; the hematopoietic growth factor receptors; the cytokine receptors; the G-protein-linked receptors; the cell surface receptors, *etc*. Such peptides typically comprise about 150 amino acid residues or less and, more preferably, about 100 amino acid residues or less (*e.g.,* ∼10-12kDa). Hydrophilic polymers suitable for use in the present invention include, but are not limited to, polyalkylethers as exemplified by polyethylene glycol and polypropylene glycol, polylactic acid, polyglycolic acid, polyoxyalkenes, polyvinylalcohol, polyvinylpyrrolidone, cellulose and cellulose derivatives, dextran and dextran derivatives, etc. Generally, such hydrophilic polymers have an average molecular weight ranging from about 500 to about 100,000 daltons, more preferably from about 2,000 to about 40,000 daltons and, even more preferably, from about 5,000 to about 20,000 daltons. In preferred embodiments, such hydrophilic polymers have an average molecular weights of about 5,000 daltons, 10,000 daltons and 20,000 daltons. The peptide compounds according to this embodiment can be derivatized with using the methods described above and in the cited references.

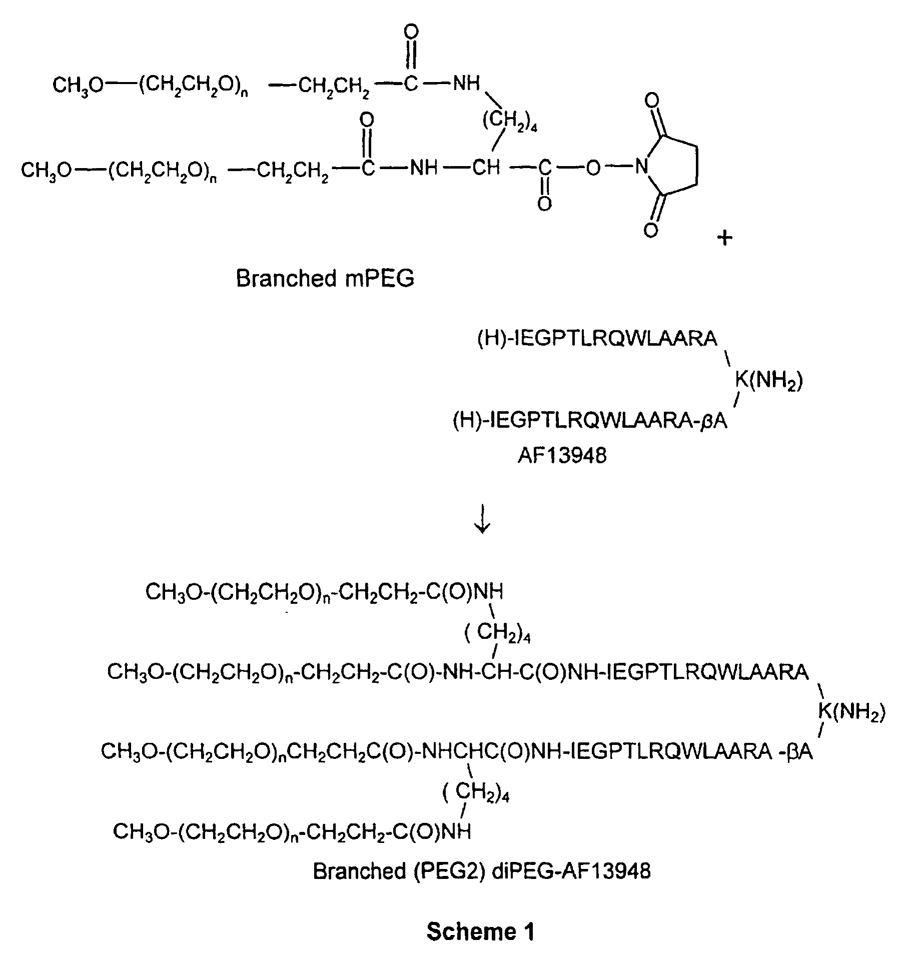

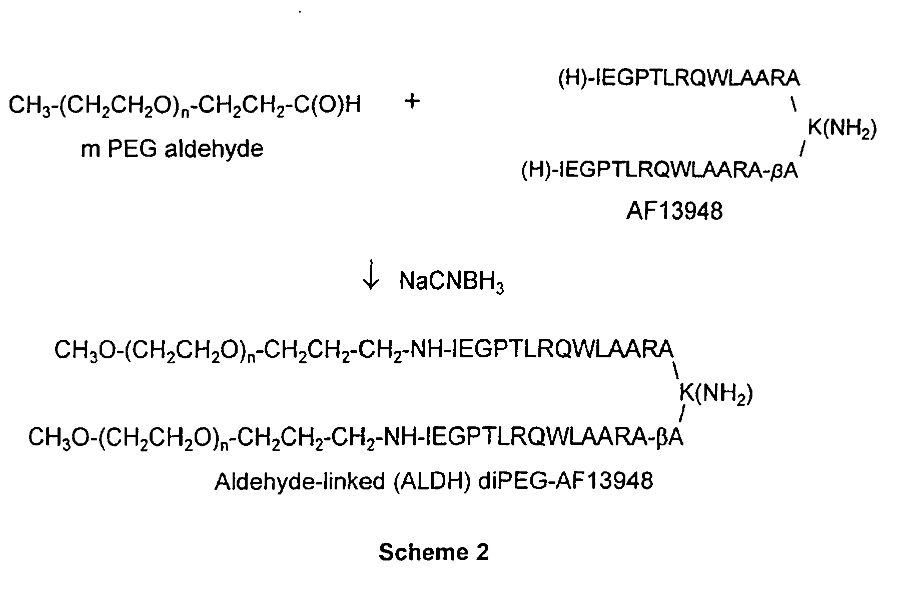

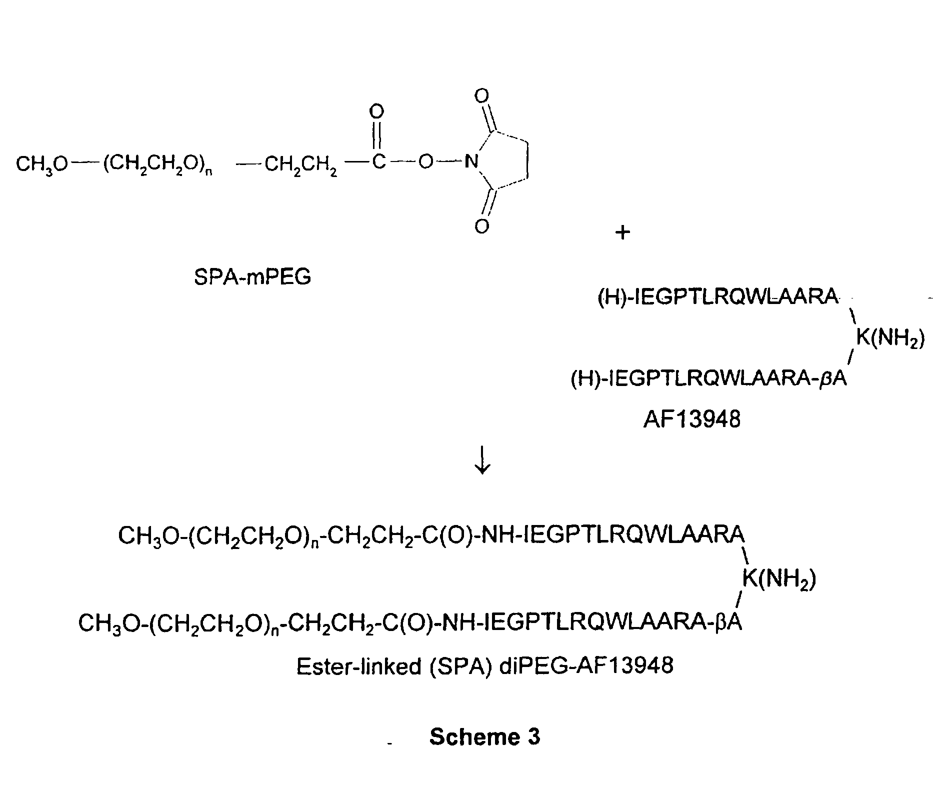

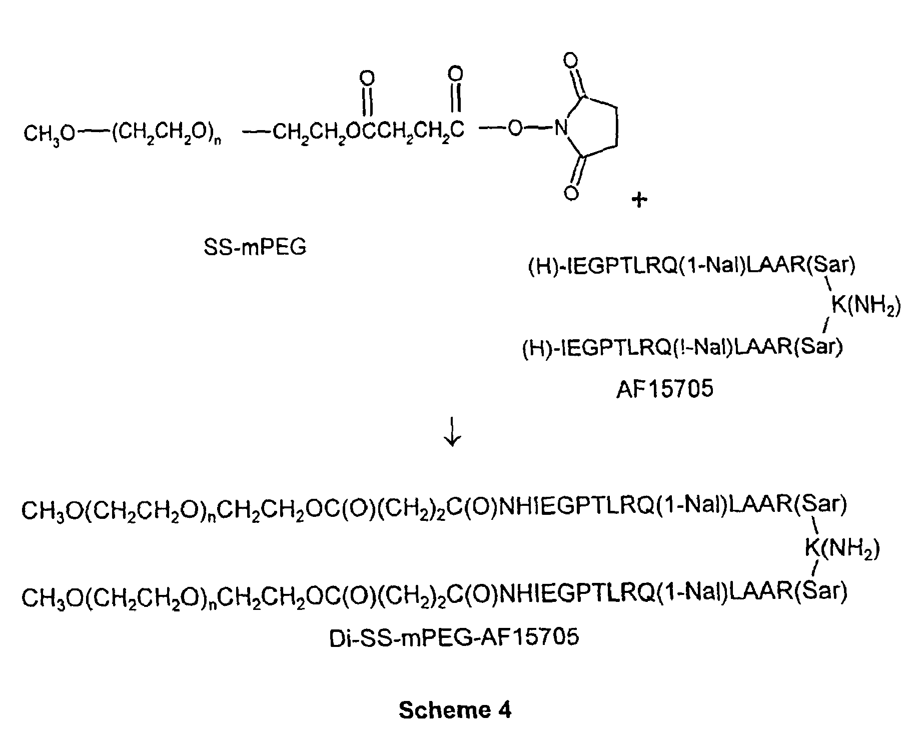

### DISULFIDE BOND FORMATION

The compound of the present invention 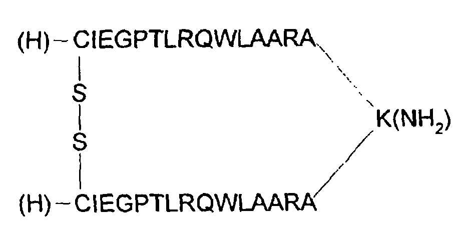 has an intramolecular disulfide bond between the thiol groups of the cysteines. This compound can be made via an intramolecular displacement, using methods known in the art (see, *e.g*., Frank A. Robey, Meth. in Mol. Bio., 35(6):73-90 (1990).

According to a further aspect, the present invention provides a process for preparing a physiologically active, substantially non-immunogenic water soluble polypeptide composition from a polypeptide originally having physiological activity comprising the steps of:
(a) activating at least one terminal carbon atom bearing a hydroxy group of a polymer having a molecular weight of from about 500 to about 20,000 daltons, wherein said polymer is unsubstituted or substituted by alkoxy or alkyl groups, said alkoxy or alkyl groups possessing less than 5 carbon atoms; and
(b) reacting a physiologically active polypeptide of formula (I) with from 10 to 100 moles of said activated polymer per mole of polypeptide by coupling said polypeptide to the reactive terminal group of said polymer to provide said physiologically active, substantially non-immunogenic water soluble polypeptide composition. Preferably the polymer is polyethylene glycol. Preferably the coupling agent is cyanuric chloride.

### UTILITY

The compounds of the invention are useful *in vitro* as unique tools for understanding the biological role of TPO, including the evaluation of the many factors thought to influence, and be influenced by, the production of TPO and the receptor binding process.

The compounds are also useful as competitive binders in assays to screen for new TPO receptor agonists. In such assay embodiments, the compounds of the invention can be used without modification or can be modified in a variety of ways; for example, by labeling, such as covalently or non-covalently joining a moiety which directly or indirectly provides a detectable signal. In any of these assays, the materials thereto can be labeled either directly or indirectly. Possibilities for direct labeling include label groups such as: radiolabels such as ¹²⁵I, enzymes (US Patent 3,645,090) such as peroxidase and alkaline phosphatase, and fluorescent labels (U.S. Patent No. 3,940,475) capable of monitoring the change in fluorescence intensity, wavelength shift, or fluorescence polarization. Possibilities for indirect labeling include biotinylation of one constituent followed by binding to avidin coupled to one of the above label groups. The compounds may also include spacers or linkers in cases where the compounds are to be attached to a solid support.

Moreover, based on their ability to bind to the TPO receptor, the peptides of the present invention can be used as reagents for detecting TPO receptors on living cells, fixed cells, in biological fluids, in tissue homogenates, in purified, natural biological materials, etc. For example, by labelling such peptides, one can identify cells having TPO-R on their surfaces. In addition, based on their ability to bind the TPO receptor, the peptides of the present invention can be used in *in situ* staining, FACS (fluorescence-activated cell sorting), Western blotting, ELISA, etc. In addition, based on their ability to bind to the TPO receptor, the peptides of the present invention can be used in receptor purification, or in purifying cells expressing TPO receptors on the cell surface (or inside permeabilized cells).

The compounds of the present invention can also be utilized as commercial reagents for various medical research and diagnostic uses. Such uses include but are not limited to: (1) use as a calibration standard for quantitating the activities of candidate TPO agonists in a variety of functional assays; (2) use to maintain the proliferation and growth of TPO-dependent cell lines; (3) use in structural analysis of the TPO-receptor through co-crystallization; (4) use to investigate the mechanism of TPO signal transduction/ receptor activation; and (5) other research and diagnostic applications wherein the TPO-receptor is preferably activated or such activation is conveniently calibrated against a known quantity of a TPO agonist, and the like.

The compounds of the present invention can be used for the *in vitro* expansion of megakaryocytes and their committed progenitors, both in conjunction with additional cytokines or on their own. See, e.g., DiGiusto, et al., PCT Publication No. 95/05843, which is incorporated herein by reference. Chemotherapy and radiation therapies cause thrombocytopenia by killing the rapidly dividing, more mature population of megakaryocytes. However, these therapeutic treatments can also reduce the number and viability of the immature, less mitotically active megakaryocyte precursor cells. Thus, amelioration of the thrombocytopenia by TPO or the compounds of the present invention can be hastened by infusing patients post chemotherapy or radiation therapy with a population of his or her own cells enriched for megakaryocytes and immature precursors by *in vitro* culture.

The compounds of the invention can also be administered to warm blooded animals, including humans, to activate the TPO-R *in vivo.* Thus, the present invention encompasses methods for therapeutic treatment of TPO related disorders that comprise administering a compound of the invention in amounts sufficient to mimic the effect of TPO on TPO-R *in vivo.* For example, the peptides and compounds of the invention can be administered to treat a variety of hematological disorders, as defined hereinbefore.

In some embodiments of the invention, TPO antagonists are preferably first administered to patients undergoing chemotherapy or radiation therapy, followed by administration of the tpo agonists of the invention.

The activity of the compounds of the present invention can be evaluated either *in vitro* or *in vivo* in one of the numerous models described in McDonald, Am. J. of Pediatric HematologylOncology, 14:8-21 (1992), which is incorporated herein by reference.

According to one embodiment, the compositions of the present invention are useful for treating thrombocytopenia associated with bone marrow transfusions, radiation therapy, or chemotherapy. The compounds typically will be administered prophylactically prior to chemotherapy, radiation therapy, or bone marrow transplant or after such exposure.

Accordingly, the present invention also provides pharmaceutical compositions comprising, as an active ingredient, at least one of the peptides or peptide mimetics of the invention in association with a pharmaceutical carrier or diluent. The compounds of this invention can be administered by oral, pulmonary, parental (intramuscular, intraperitoneal, intravenous (IV) or subcutaneous injection), inhalation (via a fine powder formulation), transdermal, nasal, vaginal, rectal, or sublingual routes of administration and can be formulated in dosage forms appropriate for each route of administration. See, *e.g.,* Bernstein, et al., PCT Patent Publication No. WO 93/25221; Pitt, et al., PCT Patent Publication No. WO 94/17784; and Pitt, *et al.,* European Patent Application 613,683, each of which is incorporated herein by reference.

Solid dosage forms for oral administration include capsules, tablets, pills, powders, and granules. In such solid dosage forms, the active compound is admixed with at least one inert pharmaceutically acceptable carrier such as sucrose, lactose, or starch. Such dosage forms can also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration include pharmaceutically acceptable emulsions, solutions, suspensions, syrups, with the elixirs containing inert diluents commonly used in the art, such as water. Besides such inert diluents, compositions can also include adjuvants, such as wetting agents, emulsifying and suspending agents, and sweetening, flavoring, and perfuming agents.

Preparations according to this invention for parental administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, bacteriostatic and dispersing agents, antoxidants, buffers, suspending agents, bulking agents and cryoprotectants. They may be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

Alternatively, the compounds may be presented as lyophilized solids for reconstitution with water (for injection), saline or dextrose solutions. Such formulations are normally presented in unit dosage forms such as vials, ampoules or disposable injection devices. They may also be presented in multi-dose forms such as a bottle from which the appropriate dose may be withdrawn. All such formulations should be sterile.

Compositions for rectal or vaginal administration are preferably suppositories which may contain, in addition to the active substance, excipients such as cocoa butter or a suppository wax. Compositions for nasal or sublingual administration are also prepared with standard excipients well known in the art.

The compositions containing the compounds can be administered for prophylactic and/or therapeutic treatments. In therapeutic applications, compositions are administered to a patient already suffering from a disease, as described above, in an amount sufficient to cure or at least partially arrest the symptoms of the disease and its complications. An amount adequate to accomplish this is defined as "therapeutically effective dose". Amounts effective for this use will depend on the severity of the disease and the weight and general state of the patient.

The compositions of the invention can also be microencapsulated by, for example, the method of Tice and Bibi (in Treatise on Controlled Drug Delivery, ed. A. Kydonieus, Marcel Dekker, N.Y. (1992), pp. 315-339).

In prophylactic applications, compositions containing the compounds of the invention are administered to a patient susceptible to or otherwise at risk of a particular disease. Such an amount is defined to be a "prophylactically effective dose". In this use, the precise amounts again depend on the patient's state of health and weight.

The quantities of the TPO agonist necessary for effective therapy will depend upon many different factors, including means of administration, target site, physiological state of the patient, and other medicants administered. Thus, treatment dosages should be titrated to optimize safety and efficacy. Typically, dosages used *in vitro* may provide useful guidance in the amounts useful for in situ administration of these reagents. Animal testing of effective doses for treatment of particular disorders will provide further predictive indication of human dosage. Various considerations are described, e.g., in Gilman, et al. (eds), Goodman and Gilman's: The Pharmacological Basis of Therapeutics, 8th ed., Pergamon Press (1990); and Remington's Pharmaceutical Sciences, 7th Ed., Mack Publishing Co., Easton, Penn. (1985); each of which is hereby incorporated by reference.

The peptides and peptide mimetics of this invention are effective in treating TPO mediated conditions when administered at a dosage range of from about 0.001 mg to about 10 mg/kg of body weight per day. The specific dose employed is regulated by the particular condition being treated, the route of administration as well as by the judgement of the attending clinician depending upon factors such as the severity of the condition, the age and general condition of the patient, and the like.

A suitable pharmaceutical parenteral preparation for administrations to humans will preferable contain 1-50 mg/ml of the compound of formula (I), or a pharmaceutically acceptable derivative therof, in solution or multiples therof for multi-dose vials.

A preferred formulation is a solution of the compound of formula (I) in water, saline, or dextrose solution. Particularly preferred solutions have a pH of about 4.0 - 5.0.

According to a further aspect the invention provides a physiologically active, substantially non-immunogenic water soluble polypeptide composition comprising a compound of formula (I) coupled with a coupling agent to at least one polymer having a molecular weight of between about 500 to about 20,000 daltons selected from the group consisting of polyethylene glycol and polypropylene glycol, wherein said polymer is unsubstituted or substituted by alkoxy or alkyl groups, said alkoxy or alkyl groups possessing less than 5 carbon atoms. Preferably said polymer has a molecular weight of about 750 to about 15,000 daltons, more preferably of about 5,000 to about 10,000 daltons. Preferably said polymer is polyethylene glycol.

Although only preferred embodiments of the invention are specifically described above, it will be appreciated that modifications and variations of the invention are possible without departing from the spirit and intended scope of the invention.

### EXAMPLE 1

### SOLID PHASE PEPTIDE SYNTHESIS

Various peptides of the invention were synthesized using the Merrifield solid phase synthesis techniques (see Steward and Young, Solid Phase Peptide Synthesis, 2d. edition, Pierce Chemical, Rockford, IL (1984) and Merrifield, J. Am. Chem. Soc., 85:2149 (1963)) either manually or using an Applied Biosystems Inc. Model 431A or 433A peptide synthesizer. The peptides were assembled using standard protocols of the Applied Biosystems Inc. Synth AssistÔ 1.0.0 or Synth AssistÔ 2.0.2. Unless otherwise noted in the examples, each coupling was performed for 2x30 min. with HBTU (2-(1H-benzatriazol-1-yl)-1,1,3,3-tetramethyluronium hexafluoro-phosphate) and HOBt (1-hydroxybenzotriazole).

The resin used was HMP resin (p-hydroxymethyl phenoxymethyl)polystyrene resin or PAL (Milligen/Biosearch), which is a cross-linked polystyrene resin with 5-(4'-Fmoc-aminomethyl-3,5'-dimethyoxyphenoxy) valeric acid as a linker. Use of PAL resin results in a carboxyl terminal amide functionality upon cleavage of the peptide from the resin. Upon cleavage, the HMP resin produces a carboxylic acid moiety at the C-terminus of the final product. Most reagents, resins, and protected amino acids (free or on the resin) were purchased from Millipore or Applied Biosystems Inc.

The Fmoc group was used for amino protection during the coupling procedure. Primary amine protection on amino acids was achieved with Fmoc and side chain protection groups were t-butyl for serine, glutamic acid, and threonine; trityl for glutamine; Pmc (2,2,5,7,8-pentamethylchroman-6-sulfonyl) for arginine; N-t-butyloxycarbonyl for tryptophan; and S-trityl for cysteine.

Removal of the peptides from the resin and simultaneous deprotection of the side chain functions were achieved by treatment with reagent K or slight modifications of it. Altematively, in the synthesis of those peptides, with an amidated carboxyl terminus, the fully assembled peptide was cleaved with a mixture of 90% trifluoroacetic acid, 5% ethanedithiol, and 5% water, initially at 4°C, and gradually increasing to room temperature. The deprotected peptides were precipitated with diethyl ether. In all cases, purification was by preparative, reverse-phase, high performance liquid chromatography on a C₁₈ bonded silica gel column with a gradient of acetonitrile/water containing 0.1% trifluoroacetic acid. The homogeneous peptides were characterized by Fast Atom Bombardment mass spectrometry or electrospray mass spectrometry and amino acid analysis when applicable.

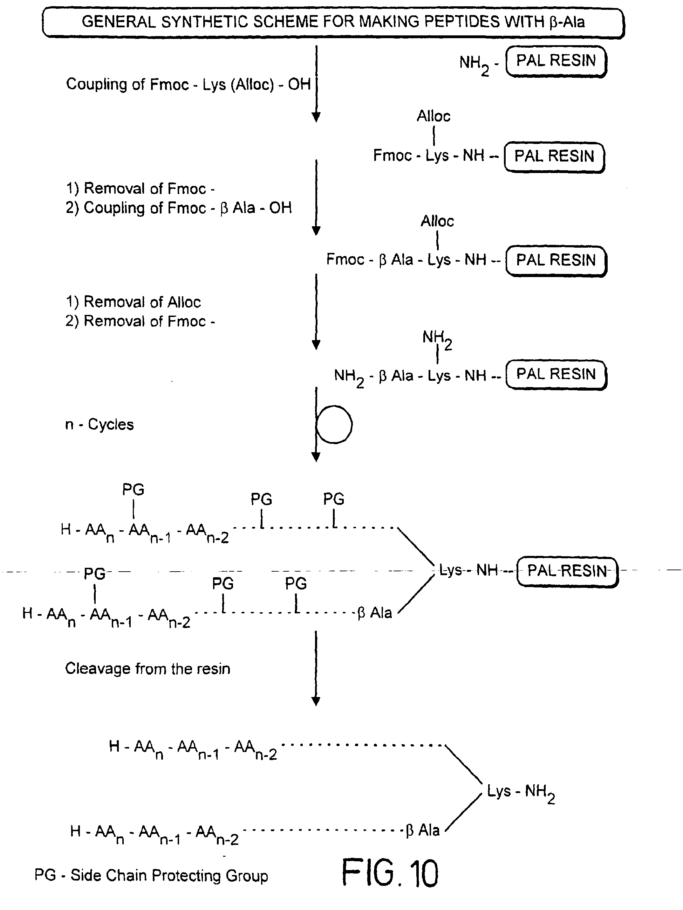

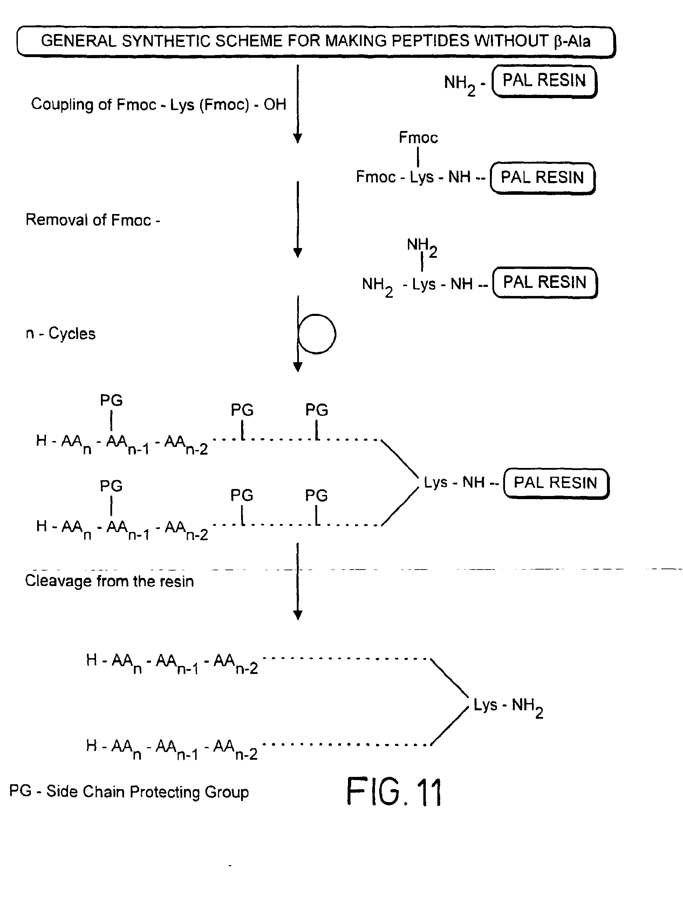

In a preferred embodiment, the peptides of this invention are dimerized using standard synthetic procedures known to and used by those of skill in the art. Exemplar synthetic schemes for preparing the dimer peptide compounds of this invention are set forth in Figures 10 and 11. Following these synthetic schemes, those of skill in the art can readily prepare dimer peptide compounds in accordance with the present invention. In addition, it will be readily apparent to those of skill in the art that the dimeric subunits can readily be linked using methodologies and linkers other than those described in Figures 10 and 11.

### A. Synthesis of AF15705

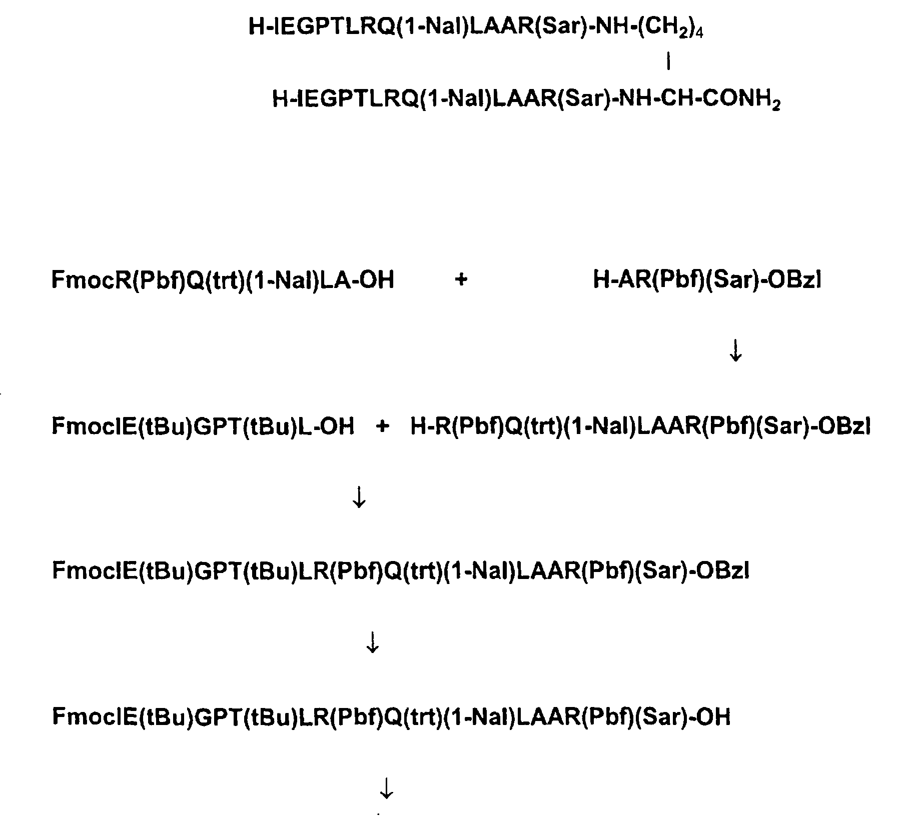

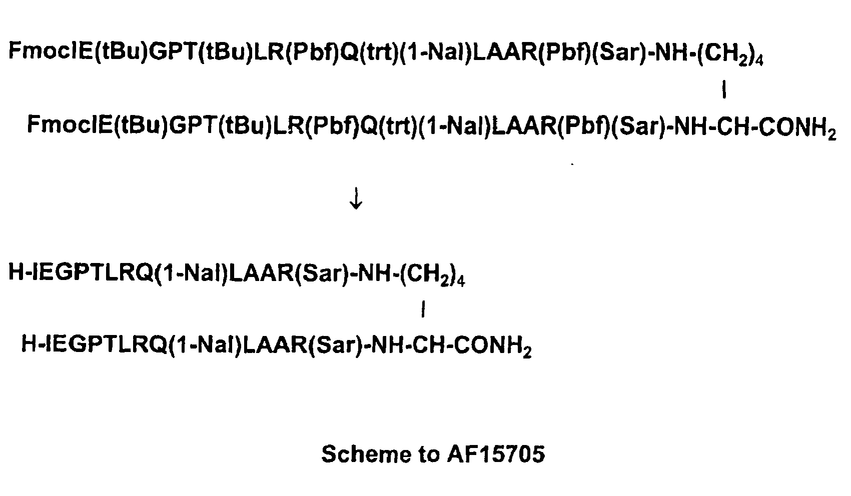

### Synthesis of FmoclE(tBu)GPT(tBu)L-OH

A 1 L peptide chamber was charged with Fmoc-Leu-HMPB-BHA [4-(4-hydroxymethyl-3-methoxyphenoxy)-butyric acid benzhydrylamine] resin (35g, 18.9 mmol) and 300 mL of 1-methyl-2-pyrrolidinone (NMP). The resin was conditioned in the solvent with nitrogen agitation for around 30 minutes to swell the beads. Fmoc (9-fluorenylmethyloxycarbonyl) removal from the terminal amine was accomplished using 2 x 250 mL of a 30% solution of piperidine in NMP for 10 minutes each. The resin was then washed free of Fmoc by-products (dibenzofulvene and its piperidine adduct) with 6 x 300 mL NMP, as determined by a negative chloranil test. Fmoc-Thr(tBu)-OH (15.0g, 2 eq) and 1-hydroxybenzotriazole (HOBT) (5.8g, 2 eq) were dissolved in 250 mL NMP at room temperature. The solution was chilled in an ice bath to 0-5 °C. Diisopropylethylamine (DIPEA) (8.2mL, 2.5 eq) was added and stirred for 3-5 minutes. *O*-Benzotriazol-1-yl-N,N,N',N'-tetramethyluronium hexafluorophosphate (HBTU) (14.3g, 2 eq) and 75 mL dichloromethane (DCM) were added and stirred to dissolve, around 5 minutes. The solution of activated acid was loaded onto the drained resin and agitated with N₂ bubbling for 1 hr. Coupling completion was monitored with the ninhydrin test. The resin was drained and washed with 3 x 300 mL NMP.

The cycle was then repeated for subsequent mers of the peptide fragment using: Fmoc-Pro-OH (12.8 g), Fmoc-Gly-OH (11.2 g), Fmoc-Glu(OtBu)-OH (16.8 g), and Fmoc-Ile-OH (13.4 g). Following the final coupling reaction, the resin was washed 4 x 300 mL NMP, then 4 x 300 mL DCM.

The peptide was cleaved from the resin using 1% trifluoroacetic acid in DCM. Fractions were collected and analyzed by HPLC for product content. Four 300 mL fractions, four 500 mL fractions, then six 250 mL fractions were collected and analyzed. Fractions 3-9 were adjusted to pH ∼3-4 with pyridine, then combined, and concentrated to near-dryness on the rotary evaporator. Around 200 mL of ethanol was introduced to the flask and the concentration was continued to remove residual DCM. The resulting partial solution was heated on a steam bath to obtain complete solution. The solution was chilled in an ice bath to 0-5 °C and 400 mL of water was added with vigorous stirring. The thick product slurry was stirred in the ice bath for - 1 hr, filtered, and washed with 100 mL of water. The product was air dried on the funnel by pulling a stream of air through the funnel overnight to give 18.8 g (19.5 mmol) of Fmoc-Ile-Glu(OtBu)-Gly-Pro-Thr(tBu)-Leu-OH in quantitative yield and 98.3 area % HPLC purity.

### Synthesis of FmocR(Pbf)Q(trt)(1-Nal)LA-OH

A 1 L peptide chamber was charged with HMPB-BHA resin (35g, 30.8 mmol) and 300 mL of 1-methyl-2-pyrrolidinone (NMP). The resin was conditioned in the solvent with nitrogen agitation for around 30 minutes to swell the beads. Meanwhile Fmoc-Ala-OH (28.8g, 3 eq) was dissolved in 200 mL NMP at room temperature. The solution was chilled in a methanol/water/ice bath to -5°C to 0°C then dimethylaminopyridine (DMAP) (750mg, 0.2 eq) was added and allowed to dissolve. Diisoproplycarbodiimide (DIC) (14.3 mL, 3 eq) and 100 mL of dichloromethane (DCM) were added and the solution was stirred at around 0°C for 20-30 minutes. A bright yellow solution was obtained. The resin was drained and the solution of activated amino acid was charged to the chamber. The mixture was agitated with N₂ bubbling for 5hrs. The resin was drained and washed with 3 x 300 mL NMP. A sample was removed, washed well with NMP and DCM, and dried overnight. The resin was end capped with a solution of benzoic anhydride (20.9 g, 3 eq) and 7.5 mL (3 eq) of pyridine in 250 mL NMP. The slurry was agitated at room temperature for 1.5 hrs. The resin was drained and washed with 3 x 300 mL NMP. Fmoc (9-fluorenyl-methyloxycarbonyl) removal from the terminal amine was accomplished using 2 x 250 mL of a 30% solution of piperidine in NMP for 10 minutes each. The resin was then washed free of Fmoc by-products (dibenzofulvene and its piperidine adduct) with 6 x 300 mL NMP as determined by a negative chloranil test.

Fmoc-Leu-OH (21.8g, 2 eq) and 1-hydroxybenzotriazole (HOBT) (9.4g, 2 eq) were dissolved in 225 mL NMP at room temperature. The solution was chilled in an ice bath to 0-5°C. Diisopropylethylamine (DIPEA) (13.4 mL, 2.5 eq) was added and stirred for 3-5 minutes. *O*-Benzotriazol-1-yl-N,N,N',N'-tetramethyluronium hexafluorophosphate (HBTU) (23.4g, 2 eq) and 75 mL DCM were added and stirred to dissolve, around 5 minutes. The solution of activated acid was loaded onto the drained resin and agitated with N₂ bubbling for 1 hr. Coupling completion was monitored with the ninhydrin test. The resin was drained and washed with 3 x 300 mL NMP.

The cycle was then repeated for subsequent mers of the peptide fragment using: Fmoc-(1-Nal)-OH (27.0 g), Fmoc-Gln(trt)-OH (37.6 g), and Fmoc-Arg(Pbf)-OH (40.0 g). Following the final coupling reaction, the resin was washed 4 x 300 mL NMP, then 4 x 300 mL DCM.

The peptide was cleaved from the resin using 1% trifluoroacetic acid in DCM. Fractions were collected and analyzed by HPLC for product content. Four 200 mL fractions, three 500 mL fractions, then five 250 mL fractions were collected and analyzed. Fractions 5-11 were adjusted to pH ∼3-4 with pyridine, then combined, and concentrated to near-dryness on the rotary evaporator. Around 200 mL of ethanol was introduced to the flask and the concentration was continued to remove residual DCM. The resulting partial solution was heated on a steam bath to obtain complete solution. The solution was chilled in an ice bath to 0-5°C and 200 mL of 0.1 N HCl was added followed by 200 mL of water. The thick product slurry was stirred in the ice bath for - 1 hr, filtered, and washed with 100 mL of water. The product was air dried on the funnel by pulling a stream of air through the funnel overnight to give Fmoc-Arg(Pbf)-Gln(trt)-(1-Nal)-Leu-Ala-OH (31.9g, 22.8 mmol) in 74% yield from the resin (>95% yield from the assayed resin) in 94.8 area % HPLC purity.

### Synthesis of H-AR(Pbf)(Sar)-OBzl

FmocArg(Pbf)-OH (3 eq), dimethylaminopyridine (0.3 eq), and diisopropyl carbodiimide (3 eq) were stirred to dissolve in 1-methyl-2-pyrrolidinone (NMP, 10 vol) at room temperature. The solution was added to the [4-(4-hydroxymethyl-3-methoxyphenoxy)-butyric acid](HMPB) resin in a peptide chamber and agitated with N₂ bubbling for 3-6 hrs. Loading efficiency was determined by quantitative HPLC analysis of a cleavage sample. The resin-bound FmocArg(Pbf) was exposed to 30% piperidine/NMP(2 x10 vol) for 10-15 minutes to deprotect the terminal amine. The resin was washed with NMP (60-80 vol) to a negative chloranil test for piperidine. FmocAla-OH (2 eq) was preactivated in NMP(5-10 vol) at 0-5°C using *O*-Benzotriazol-1-yl-N,N,N',N'-tetramethyluronium hexafluorophosphate (HBTU, 2 eq) and 1-Hydroxybenzotriazole (HOBT, 2 eq), and diisopropylethylamine (DIPEA, 2.1 eq), then transferred to the resin mixture and stirred with a nitrogen purge for 30-90 minutes. Coupling efficiency was monitored with the ninhydrin test. The resin was washed with NMP (25-30 vol) and the coupling cycle was repeated to provide the fully side-chain protected resin-bound peptide. The resin is washed free of NMP with methylene chloride (DCM, 40-60 vol) prior to cleavage of the peptide. The side-chain protected fragment was removed from the resin with several 1% trifluoroacetic acid /DCM (20 vol) rinses. The resin turned a deep violet color when the peptide was released. The cleavage fractions were monitored by HPLC, then neutralized with pyridine and concentrated to a solid residue which underwent further purification. To the solids was added 300 mL of water. The pH of the solution was adjusted to 2 with 0.1N HCl (100 mL). The suspension was washed with ethyl acetate (2 x 300 mL). The combined ethyl acetate extracts were washed with saturated aqueous sodium chloride (150 mL), dried over MgSO₄ and concentrated to a foam. The foam was crushed, and triturated with methyl t-butyl ether (MTBE 100 mL). The resulting solid was collected and dried to give FmocAR(Pbf)-OH in ∼100% yield from the resin.

A solution of FmocAR(Pbf)-OH (1 eq), SarOBzl, tosylate salt (1 eq) , HOAT (1.2 eq) and DIPEA (2.5 eq) in DMF (13 vol) was cooled to 0°C and HBTU (1.2 eq) was added. The solution was stirred at 0°C for 10 minutes then warmed to ambient temperature and stirred for 40 minutes. To precipitate the peptide, a 1:1 solution of brine/water (40 vol) was added. The solid was collected, dissolved in methanol (8 vol) and precipitated with water (10 vol). The solvent was decanted from the sticky semi-solid and the semi-solid was dried *in vacuo* to a foam. The foam was crushed and triturated with MTBE (8 vol). The solvent was decanted and the solid dried. Yield 15.2g, 92% from the resin, 99 area % by HPLC. The product was dissolved in 9:1 THF/piperidine (10 vol) and stirred at ambient temperature for 50 minutes. To precipitate the peptide MTBE (26 vol) and hexane (33 vol) was added. The solvent was decanted from the semi-solid and the semi-solid (which gums on exposure to air) was dried to a foam *in vacuo.* The foam was crushed, triturated with MTBE (10 vol) and the solvent decanted. The trituration was repeated twice and the product dried. Yield 93%, HPLC purity, 95 area %.

### Alternate Solution Synthesis of H-AR(Pbf)(Sar)-OBzl using t-Boc protection

Boc-Arg(Pbf)-OH.DCHA (4.96 g) was partitioned between 50 ml of ethylacetate and two 40 ml portions of 0.25 M citric acid. The organic phase was washed with two 30 ml volumes of water, 30 ml of brine, dried over magnesium sulfate and evaporated to a foam. The foam, Sar-OBzl tosylate salt (2.71 g) and HOAt (0.98 g) were dissolved in 50 ml of methylene chloride. The solution was chilled in an ice-bath and DCC (1.50 g) and DIPEA (1.8 ml) were added. The reaction mixture was stirred in the cold for 1/2 hr and then at room temperature for 18 hr. The mixture was filtered to remove precipitated DCU, and the methylene chloride was removed by evaporation under vacuum. The residual oil was dissolved in ethyl acetate and washed copiously with 0.1 M citric acid, water, sodium bicarbonate solution, water and brine. The washed solution was dried over magnesium sulfate, filtered and evaporated to a glassy foam (4.15 g). Boc-Arg(Pbf)-Sar-OBzl (3.80 g) was dissolved in 20 ml of methylene chloride, to this solution was added a solution of HCl in MTBE (10 ml). The mixture was stirred for at least an hour until no starting material was detected by TLC analysis. The solution was evaporated to dryness and held over night under high vacuum. The foam, Boc-Ala-OH (1.14 g) and HOAt (0.81 g) were dissolved in 20 ml of methylene chloride. The solution was chilled in an ice-bath and treated with DCC (1.24 g) and DIPEA (1.5 ml). The reaction mixture was stirred in the cold for half an hour, and then at room temperature for 1.5 hr.The precipitated DCU was removed by filtration, the methylene chloride was replaced with 50 ml of ethyl acetate and the solution was washed with citric acid, water, bicarbonate solution, water and brine. The washed organic solution was dried over magnesium sulfate and evaporated to a foam (3.66 g). The foam was treated with HCl, as described above for the dipeptide, to give H-Ala-Arg(Pbf)-Sar-OBzl as the hydrochloride salt.

### Synthesis of H-R(Pbf)Q(trt)(1-Nal)LAAR(Pbf)(Sar)-OBzl.

A solution of FmocR(Pbf)Q(trt)(1-Nal)LA-OH (1 eq), H-Ala-Arg(Pbf)-Sar-OBzl (1 eq), HOAT (1.2 eq) and DIPEA (2.2 eq) in DMF (12 vol) was cooled to 0°C and HBTU (1.2 eq) was added. The solution was warmed to ambient temperature and stirred for 50 min. To the solution was added water (25 vol) to precipitate the peptide. The solid was collected by filtration. The still-damp solid was dissolved in hot ethanol (15 vol), then stirred while cooling to 30°C. The resulting solid was collected by filtration and dried to yield the product in 75% yield. HPLC purity 91 area %. The solid was dissolved in 9:1 THF/piperidine (15 vol) and stirred at ambient temperature for 50 minutes. To precipitate the peptide, MTBE (15 vol) was added. The solid was collected by filtration, then triturated with warm ethanol (8 vol). After cooling to ambient temperature with stirring, the solid was collected and dried. Yield, 92%. HPLC purity, 93 area %.

### Synthesis of

### FmoclE(tBu)GPT(tBu)LR(Pbf)Q(trt)(1-Nal)LAAR(Pbf)(Sar)-OBzl

A solution of FmoclE(tBu)GPT(tBu)L-OH (1 eq), H-R(Pbf)Q(trt)(1-Nal)LAAR(Pbf)(Sar)-OBzl (1 eq), HOAT (1.2 eq) and DIPEA (2 eq) in DMF (13.5 vol) was cooled to 0°C and HBTU was added. The solution was warmed to ambient temperature and stirred for 1 hour. Water (20 vol) was added to precipitate the peptide. The solid was collected and triturated with methanol (8 vol). The solid was collected, dried and dissolved in 98/2 DCM/methanol (5 vol). The solution was loaded onto a column containing 700g of silica gel in 98/2 DCM/methanol. The column was eluted with 1.6L of 98/2 DCM/methanol, then 1 L of 97/3 DCM/methanol and 8L of 95/5 DCM/methanol. The fractions containing the product were combined and concentrated to a foam. Yield, 80.7%, HPLC purity, 85 area %.

### Synthesis of FmoclE(tBu)GPT(tBu)LR(Pbf)Q(trt)(1-Nal)LAAR(Pbf)(Sar)-OH

A solution of FmoclE(tBu)GPT(tBu)LR(Pbf)Q(trt)(1-Nal)LAAR(Pbf)(Sar)-OBzl in 8:1 DCM/ethanol (15 vol) was charged with Pd/C (0.4wt, Degussa type E101, 10% dry wt, 50% water). The reaction vessel was purged with nitrogen and evacuated three times, then placed under an atmosphere of hydrogen (balloon) and stirred at ambient temperature for 20 hours. The slurry was filtered through a tightly packed bed of celite. The filtrate was concentrated to about 2 volumes and MTBE (10 vol) was added to precipitate the peptide. The solid was collected and dried to a gray solid. Yield 96%, 85.5 area % HPLC purity.

### Synthesis of [FmoclE(tBu)GPT(tBu)LR(Pbf)Q(trt)(1-Nal)LAAR(Pbf)(Sar)]₂LysNH₂

A slurry of FmoclE(tBu)GPT(tBu)LR(Pbf)Q(trt)(1-Nal)LAAR(Pbf)(Sar)-OH (2.4 eq), (HCl)₂ LysNH₂ (1 eq), HOBT (2.5 eq) and EtPr₂N (4.8 eq) in DMF (15 vol) was stirred at ambient temperature until all the solids dissolved (5 minutes). To the solution was added HBTU (2.5 eq). After stirring for 2 hours at ambient temperature, water (30 vol) was added to precipitate the peptide. The crude peptide was collected by filtration. The still-damp solid was dissolved in hot ethanol (12.5 vol), then cooled to ambient temperature while stirring overnight. The solid was collected and dried to give the product, quantitative yield with an HPLC purity of 80 area %.

### Synthesis of AF15705

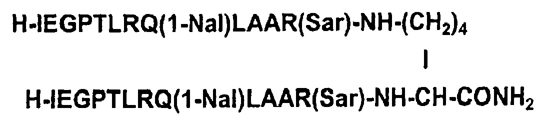

A solution of [FmoclE(tBu)GPT(tBu)LR(Pbf)Q(trt)(1-Nal)LAAR(Pbf)(Sar)]₂LysNH₂ in 9:1 NMP/piperidine (10 vol) was stirred at ambient temperature for 1-2 hours. Water (12 vol) was added to precipitate the peptide. The solid was collected, washed with water (5 vol) and dried. The dry solid was triturated with MTBE (12 vol) followed by AcCN (8 vol) to remove residual piperidine, fulvene and piperidine fulvene adduct. The solid was collected by vacuum filtration and dried. Yield 97%. A 95/5 TFA/water solution (13 vol) was purged with nitrogen, then cooled to 0°C. To this was added [HIE(tBu)GPT(tBu)LR(Pbf)Q(trt)(1-Nal)LAAR(Pbf)(Sar)]₂LysNH₂ (1 wt) as obtained above. The suspension was stirred at 0°C until all the solid dissolved (30 min), then warmed to ambient temperature and stirred for 100 minutes. The solvent volume was reduced by 50% using a rotary evaporator and then ice/water (150 vol) was added. The suspension was stirred at 0°C for 1.5 hours to allow the peptide to dissolve, then filtered through sintered glass. The glassware was washed with water (50 mL) which was then filtered and added to the product solution. The aqueous solution was frozen and lyophilized to a solid which was stored at 0°C prior to purification. The product was loaded onto a column of C18 modified silica gel (100A° pore-size, 10µ spherical particles) and eluted with a gradient of about 30% to about 35% acetonitrile in water, both solvents containing 0.1% TFA. Fractions containing the desired peptide, of at least 90% AUC were combined, concentrated and lyophilized. Yields of the purified peptide, from the fully protected precursor, are generally on the order of 10% to 25%.

Structural confirmation of peptide fragments in Example 1A leading up to the assembled peptide AF15705. Molecular weight confirmation by mass spectrometry (MS), composition by amino acid analysis (AAA), and peptide sequence information by Edman Degradation.

| **Compound #** | **MS/ID** **[m+H]⁺** | **AAA¹** | **Edman Deg²** |
|---|---|---|---|
| H-AR(Pbf)(Sar)-OBzl | 659.3 | Conforms | Conforms |
| FmocR(Pbf)Q(trt)(1-Nal)LA-OH | 1400.6 | Conforms | Conforms |
| FmoclE(tBu)GPT(tBu)L-OH | 963.5 | Conforms | Conforms |
| H-R(Pbf)Q(trt)(1-Nal)LAAR(Pbf)(Sar)-OBzl | 1818.9 | Conforms | Conforms |
| FmoclE(tBu)GPT(tBu)LR(Pbf)Q-(trt)(1-Nal)LAAR(Pbf)(Sar)-OBzl | 2763.4 | Conforms | Conforms |
| FmoclE(t8u)GPT(tBu)LR(Pbf)Q-(trt)(1-Nal)LAAR(Pbf)(Sar)-OH | 2673.4 | Conforms | Conforms |
| [FmoclE(tBu)GPT(tBu)LR(Pbf)Q-(trt)(1-Nal)LAAR(Pbf)(Sar)]₂LysNH₂ | Not Run³ | Conforms | Conforms |
| AF15705 | 3293.9 | Conforms | Conforms |

| | | | |
|---|---|---|---|
| ¹ Results are consistent with the expected AA composition. ² Results are consistent with the expected AA sequence. ³ Not run - parent ion mass exceeds range of mass spectrometer and protecting groups prevent double charge (i.e., [m+2H]²⁺) on molecule, thus no associated ions are detected. | | | |

### PEGYLATION OF THE PEPTIDES

### B. Preparation of di-PEGylated AF13948 with PEG2 branched PEG, 20,000Mw (see, Scheme 1)

AF13948 was dissolved in 100mM bicine pH 8.0 at a concentration of 10mg/ml, added to a 1.25 fold molar excess of powdered PEG2 (commercially available from Shearwater Polymers, Inc. (Huntsville, AL)) and stirred at room temperature until the reaction was complete, typically 1-2 hours. The reaction was monitored by reverse phase HPLC using a 40-65% acetonitrile gradient with a YMC ODS AQ column. When the reaction was complete, the solution was added to a second 1.25 molar excess of powdered PEG2 and the process was repeated 4 times using a total of 5 moles of PEG2 for each mole of AF13948. The solution was diluted 2 fold with PBS to reduce the viscosity and loaded onto a superdex 200 column (Pharmacia), previously equilibrated and eluted with PBS. Fractions from the size exclusion column were analyzed by reverse phase HPLC. Fractions containing di-PEG-AF13948 which eluted prior to any mono-PEG-AF13948 were pooled and stored at 5°C or lyophilized.

### C. Preparation of di-PEGylated AF13948 with SPA-mPEG 20,000Mw (see, Scheme 3)

AF13948 was dissolved in 100mM bicine pH 8.0 at a concentration of 10mg/ml, added to a 5 fold molar excess of powdered SPA-mPEG (commercially available from Shearwater Polymers, Inc. (Huntsville, AL)) and stirred at room temperature until the reaction was complete, typically 2 hours. The reaction was monitored by reverse phase (YMC ODS AQ) HPLC and when it was complete, the solution was diluted 10 fold with deionized water and loaded onto a SP-sepharose column equilibrated in 2mM sodium phosphate buffer at pH 7.0. The unreactded PEG and the NHS flowed through the column. The PEGylated AF13948 was then eluted using a 0 to 150mM NaCl gradient, with the di-PEGylated form eluting before any mono-PEGylated material. The fractions were analyzed by reverse phase HPLC and the appropriate fractions combined. The di-PEG-AF13948 was buffer exchanged into PBS and either stored at 5°C or lyophilized.

### D. Preparation of di-PEGylated AF13848 with mPEG aldehyde 20,000Mw (see, Scheme 2)

AF13948 was dissolved in 100mM sodium phosphate pH 7.0 at a concentration of 10mg/ml, added to a 6 fold molar excess of powdered PEG aldehyde (commercially available from Shearwater Polymers, Inc. (Huntsville, AL)) and 1 M sodium cyanoborohydride solution was added to give a final sodium cyanoborohydride concentration of 100mM. The solution was stirred for 18 hours at room temperature, then buffer exchanged into PBS, using a 3K (Amicon YM) ultrafiltration membrane. The solution ws diluted 2 fold with PBS to reduce the viscosity and loaded on a superdex 200 column (Pharmacia), previously equilibrated and eluted with PBS. Fractions from the size exclusion column were analyzed by reverse phase HPLC. Fractions containing di-PEG-AF13948 which eluted prior to any mono-PEG-AF13948 were pooled and stored at 5°C or lyophilized.

### E. Preparation of di-PEGylated AF15705 with SPA-mPEG 20,000Mw (di-PEG(20K) AF15705)

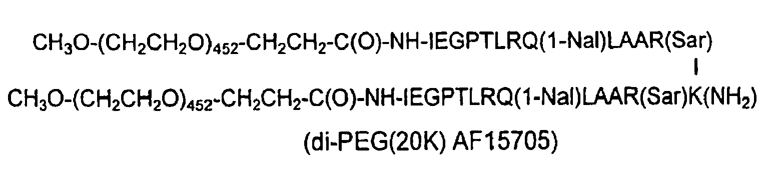

AF15705 (2.30g wt) was placed in a flask equipped with stirring and a N₂ purge and kept at room temperature. MilliQ Water (177mL) was added and the material stirred until dissolved. A 20µL protion was removed, diluted to 1mL with water and the concentration determined from a UV reading @284nm (9.36mg/ml). Sodium bicarbonate (0.2921g) was added and the solution stirred until all was dissolved (measured pH is 8). In a glove bag under N₂, four portions of M-SPA-PEG-20k (Shearwater Polymers, Inc.) were weighed into sealed bottles (each portion being 8.67g). These were placed in a sealed plastic bag and kept at 5°C until added to the reaction. One portion was added immediately after the sodium bicarbonate dissolved, the second 0.5 hour later. The third portion was added 1 hour after the second addition, and the fourth 1 hour after the third portion was added. The reaction was allowed to stir at room temperature overnight. The reaction mix was diluted nine fold with aqueous 5% EtOH and filtered through a 0.45 micron cellulose nitrate filter. This material was pumped onto a strong cation exchange column (SP Sepharose Fast Flow, 10 cm x 53 cm) at 20 mL/min. The material was eluted from the column using a gradient consisting of 90 minutes of 2mM sodium phosphate at pH 6.0, followed by a 60 minute ramp from 2mM sodium phosphate to 10mM sodium phosphate at pH 6.0, and finally, a 90 minute ramp from 10mM sodium phosphate to 100% PBS at pH 7.0. The flow rate of this chromatography was100 mL/min. the detector was set at 215nm. The column fractions were analyzed by SEC-HPLC using a G3000SWxl and G4000SWxl TSK column in series. Those fractions containing >95% product were pooled and concentrated to 33 mg/mL using an A/G Technologies UFP-30-C-6A hollow fiber cartridge. The retentate was cooled with a circulating bath at 5 deg C and was purged with N₂. The product solution was buffer exchanged into MilliQ water exchanging six retentate volumes and then further concentrated to 66 mg/mL. Twenty one grams of product were recovered following synthesis, purification and ultrafiltration. Analysis by MALDI-TOF (matrix assisted laser desorption time of flight) mass spectrometry determined that the product had a broad molecular wieght distribution centered at 46000 daltons. Tryptic digests of the product followed by either reverse phase or mixed mode HPLC, confirmed the sites of attachment of the PEG (both N-termini) and that the underlying peptide had not been degraded.
(Note: MALDI-TOF-MS shows the starting PEG to be 21.5kd rather than the advertised 20kd, therfore Mw = 21500(PEG)x2 + 3295(AF15705) = 46000).

### EXAMPLE 2

### BIOASSAYS

Bioactivity of the peptides can be measured using a thrombopoietin dependent cell proliferation assay. Murine IL-3 dependent Ba/F3 cells were transfected with full length human TPO-R. In the absence of IL-3 (WEHl-3 conditioned media), these cells are dependent on TPO for proliferation. The parental, untransfected cell line does not respond to human TPO, but remains IL-3 dependent. Bioassays have been performed on both of the above cell lines using compounds according to the invention. The cells were grown in complete RPMI-10 media, containing 10% WEHI-3 conditioned media, then washed twice in PBS, resuspended in media which lacked WEHI-3 conditioned media, and added to wells containing dilutions of peptide or TPO at 2 x 10⁴ cells/well. The cells were incubated for 48 hours at 37°C in a humidified 5% CO₂ atmosphere and metabolic activity was assayed by the reduction of MTT to formazan, with absorbance at 570 Nm measured on an ELISA plate reader. The peptides tested stimulated proliferation of TPO-R transfected Ba/F3 cells in a dose dependent manner as shown in Table 1. These peptides have no effect on the parental cell line.

### EXAMPLE 3

### BINDING AFFINITY

Binding affinities of the compounds according to the invention for TPO-R were measured in a competition binding assay. The wells of a microtiter plate were coated with 1 mg streptavidin, blocked with PBS/1% BSA, followed by 50 ng of biotinylated anti-receptor immobilizing antibody (Ab179). The wells were then treated with a 1:10 dilution of soluble TPO-R harvest. Various concentrations of test compound were mixed with a constant amount of a truncated form of TPO consisting of residues 1-156 fused to the C-terminus of maltose binding protein (MBP-TPO₁₅₆). The peptide MBP-TPO₁₅₆ mixtures were added to the TPO-R coated wells, incubated for 2 hours at 4°C and then washed with PBS. The amount of MBP-TPO₁₅₆ that was bound at equilibrium was measured by adding a rabbit anti-sera directed against MBP, followed by alkaline phosphatase conjugated goat anti-rabbit IgG. The amount of alkaline phosphatase in each well was then determined using standard methods.

The assay is conducted over a range of test compound concentrations and the results are graphed such that the y axis represents the amount of bound MBP-TPO₁₅₆ and the x axis represents the concentration of test compound. One can then determine the concentration at which the test compound will reduce by 50% (IC₅₀) the amount of MBP-TPO₁₅₆ bound to immobilized TPO-R.

### EXAMPLE 4

In this example, various substitutions, deletions and additions to the reference compound AF13948 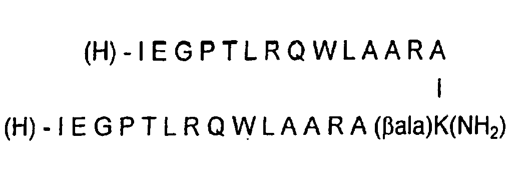 were analyzed using three assays. First, an MTT cell proliferation assay was performed as described above. Second, a microphysiometer assay was performed (Molecular Devices Corp.). Basically, in this assay the rate of acidification of the extracellular medium in response to TPO receptor stimulation by the compounds of the invention was determined. The ranges for EC₅₀ are symbolically indicated as for IC₅₀ described above. Finally, a reporter assay was performed. BaF3/TPOR cells transfected with a c-fos/luciferase reporter plasmid were starved overnight in complete RPMI-10 media containing 0.1 % WEHI-3 conditioned media, then washed two times in PBS. Cells were resuspended in media which lacked WEHI-3 conditioned media, and added to wells containing serial dilutions of peptide at 5 x 10⁵ cells/well. The cells were incubated for two hours at 37° in a humidified 5% CO₂ atmosphere, and luciferase expression was measured with a luminometer after addition of the luciferin substrate.

**Table 1**

| **Peptide Sequence** | **EC50 (pM) Cell Prolif.** | **EC50 (pM) Reporter** | **EC50 (pM) Microphys** |
|---|---|---|---|
| 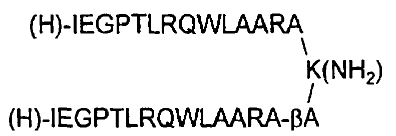 | ++ | ++ | ++ |
| 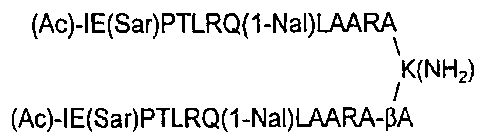 | ++ | ++ | ++ |
| 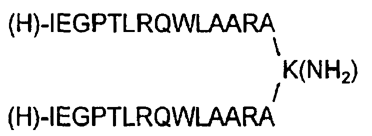 | ++ | ++ | ++ |
| 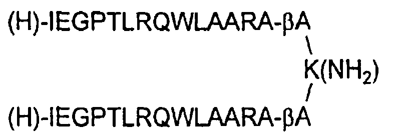 | ++ | ++ | ++ |
| 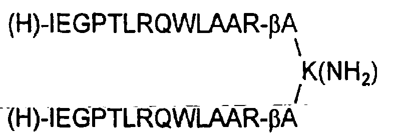 | ++ | ++ | ND |
| 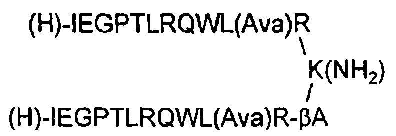 | ++ | ++ | ND |
| 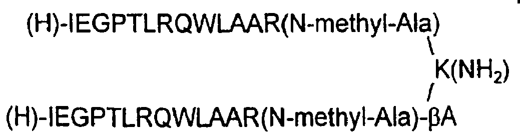 | ++ | ++ | ND |
| 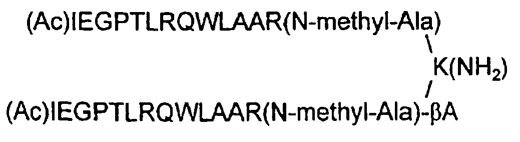 | ++ | ++ | ++ |
| 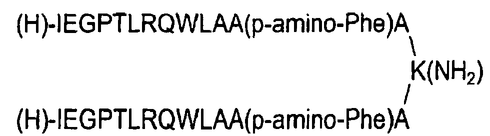 | ++ | ++ | ND |
| 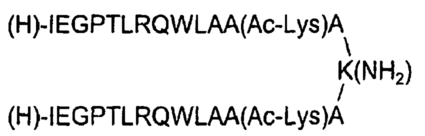 | ++ | ++ | ++ |
| 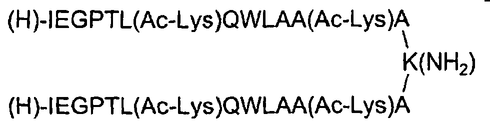 | ++ | ++ | ND |
| 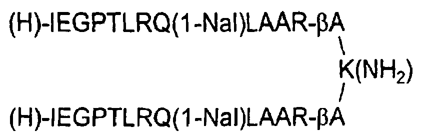 | ++ | ++ | ++ |
| 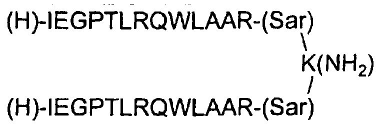 | ND | ++ | ND |
| 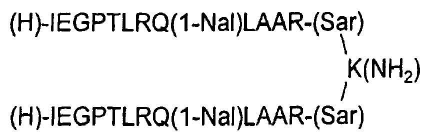 | ++ | ++ | ++ |
| 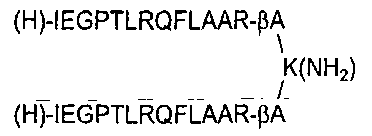 | ND | ++ | ND |
| 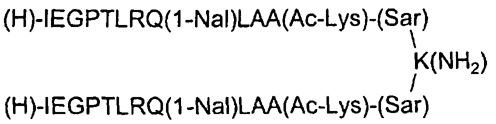 | ++ | ++ | ++ |
| 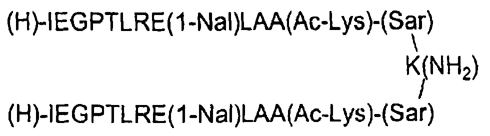 | ND | ++ | ND |
| 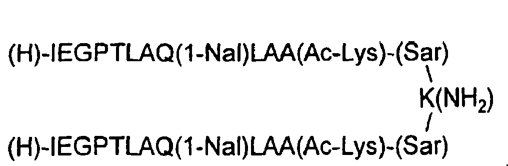 | ND | ++ | ND |
| 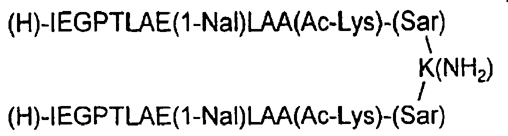 | ND | ++ | ND |
| 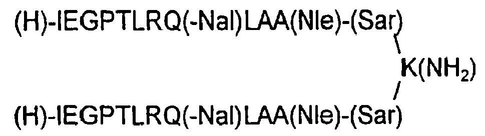 | ++ | ++ | ND |
| 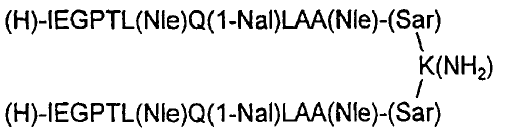 | ++ | ND | ND |

**Table 2**

| **Peptide Sequence** | **EC50 (nM) Cell Prolif.** | **IC50 (nM)** |
|---|---|---|
| 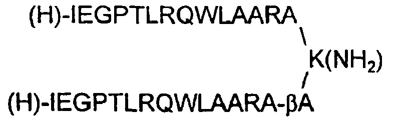 | ++ | ++ |
| 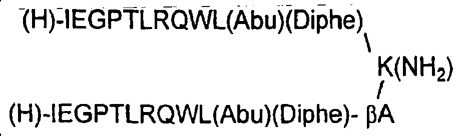 | ++ | ++ |
| 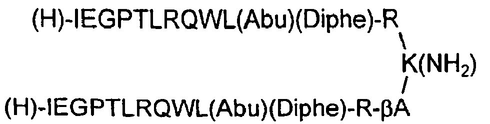 | ++ | ++ |
| 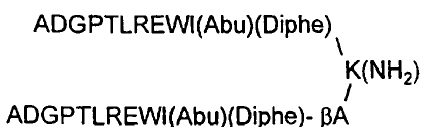 | ++ | ++ |
| 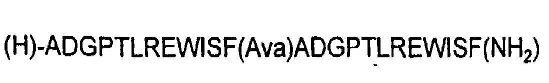 | ++ | ND |
| 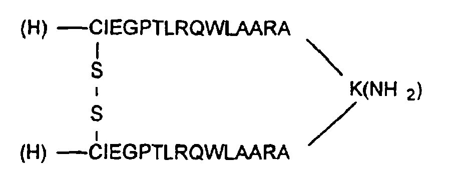 | ++ | ND |

### EXAMPLE 5

In this example, the pharmacokinetic behavior of various PEGylated peptides was determined. Pharmacokinetic behavior of compounds is an important determinant of their pharmacological activity. A key component of the pharmacokinetic profile is the persistence of the compound in the plasma of laboratory animals. This persistence is usually expressed in terms of compound concentration in plasma as a function of time after administration.

Throughout these experiments, male 20-25 g Balb/c mice were used. A volume of 200ml was injected IV or SC. The vehicle was Dulbecco's PBS; 5% DMSO; 0.1 % w/v BSA. Plasma was harvested at sacrifice using heparin as anticoagulant.

Compound concentrations were measured using a reporter cell assay. Dilutions of plasma were added to the Baf/3 TPOr/fos/lux construct 3. Luciferase expression was measured with a luminometer after addition of the luciferin substrate. The stimulatory activity of the individual plasma samples was converted to a concentration expressed as peptide equivalents of compound per volume of plasma (nM or ng/ml). This concentration was established by comparison with a standard curve constructed in the reporter assay with the parent compound.

Table 3 shows the concentrations in plasma of compounds AF13948 and the dipegylated AF13948 (polyethylene glycol (PEG) average MW=5000D) as a function of time after injection of 700µg peptide equivalents/kg. Administration of AF13948 results in activity in plasma detectable above the level present in vehicle-injected mice until 60min PI. The addition of the 5K PEG increases the concentration in plasma greater than 100-fold and extends the time it can be detected in plasma until at least 240 min PI.

**Table 3**

| Plasma concentrations of AF13948, Di-PEG(5K)-AF13948 following IV injection of 700µg peptide equivalents/kg expressed as peptide equivalents (ng/ml) | | | |
|---|---|---|---|
| time (min) | Di-PEG(5K)-AF13948 | AF13948 | vehicle |
| 10 | 361.36 | 0.80 | 0.02 |
| 30 | 91.49 | 0.26 | 0.02 |
| 60 | 54.59 | 0.11 | 0.02 |
| 120 | 16.54 | 0.04 | 0.03 |
| 240 | 11.20 | 0.02 | 0.01 |

Table 4 shows the concentrations in plasma of the compounds di-PEG(5K)-AF13948 and di-PEG(20K)-AF13948 as a function of time after injection of 500µg peptide equivalents/kg. Concentration and persistence in plasma of the 20K PEG-modified compound is greatly increased above that of di-PEG(5K)-AF13948.

**Table 4**

| Plasma concentrations of AF13948, di-PEG(5K)-AFL13948 following IV injection of 500µg peptide equivalents/kg expressed as peptide equivalents (ng/ml) | | | |
|---|---|---|---|
| time (hours) | 5K | 20K | vehicle |
| 0.5 | 54.56 | 1257.50 | 0.04 |
| 1.5 | 16.89 | 3481.31 | 0.03 |
| 4 | 2.76 | 1970.46 | 0.02 |
| 8 | 1.02 | 1158.59 | 0.02 |
| 16 | 0.17 | 259.41 | 0.03 |
| 24 | 0.13 | 710.75 | 0.04 |

Table 5 shows the plasma concentrations of compound di-PEG(20K)-AF13948 after injection of 100, 10 and 1µg peptide equivalents/kg and extends the time of observation to 120 hours PI. Concentrations observed in plasma were found to be proportional to administered doses. Administration of a single IV dose of 100µg/kg of the compound resulted in elevated plasma concentrations for at least 96 hours PI.

**Table 5**

| Plasma concentrations of di-PEG(20K)-AF13948 (nM) following IV injection of 1, 10, or 100µg peptide equivalents/kg | | | |
|---|---|---|---|
| time (hours) | dose (mg/kg) | | |
| | 100 | 10 | 1 |
| 0.08 | 291.66 | 32.89 | 2.93 |
| 0.25 | 550.82 | 45.48 | 2.63 |
| 0.5 | 1318.53 | 29.16 | 2.33 |
| 1.5 | 328.30 | 39.12 | 0.98 |
| 3 | 285.43 | 25.77 | 1.39 |
| 8 | 134.60 | 8.33 | 0.90 |
| 24 | 106.36 | 11.79 | 0.20 |
| 48 | 56.64 | 0.70 | 0.06 |
| 72 | 15.19 | 0.57 | 0.01 |
| 96 | 0.42 | 0.01 | 0.00 |
| 120 | 0.02 | 0.01 | 0.00 |

Table 6 shows the plasma concentrations following the SC and IV injection of 10µg peptide equivalents/kg of di-PEG(20K)-AF13948. SC injection of a single 10µg peptide equivalents/kg dose resulted in elevated plasma activities for 96 hours PI. Profiles of plasma concentrations achieved with these 2 routes of administration indicate the good bioavailability of SC administered di-PEG(20K)-AF13948.

**Table 6**

| **Plasma concentrations of di-PEG(20K)-AF13948 (nM) following IV and SC injection of 10µg peptide equivalents /kg** | | |
|---|---|---|
| **time** **(hours)** | **SC** | **IV** |
| 0.08 | 0.02 | 32.89 |
| 0.25 | 0.06 | 45.48 |
| 0.5 | 0.09 | 29.16 |
| 1.5 | 0.36 | 39.12 |
| 3 | 0.82 | 25.77 |
| 8 | 1.48 | 8.33 |
| 24 | 7.27 | 11.79 |
| 48 | 1.09 | 0.70 |
| 72 | 0.03 | 0.57 |
| 96 | 0.01 | 0.01 |
| 120 | 0.01 | 0.01 |

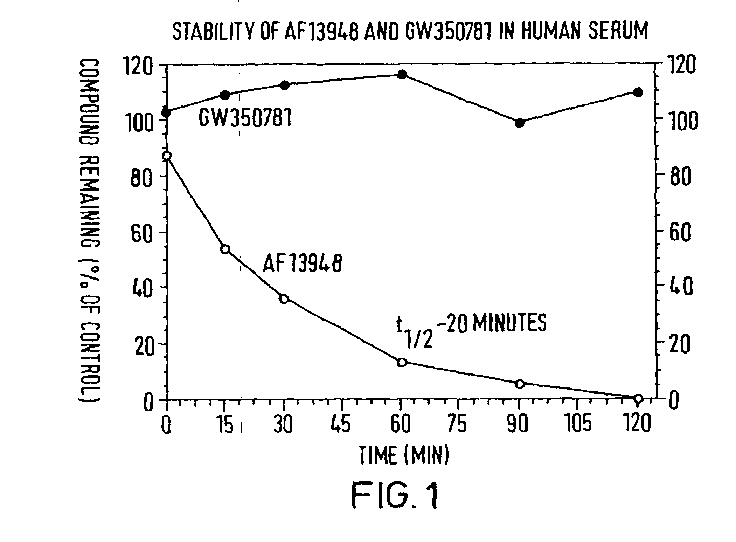

In addition, Figure 1 indicates that GW350781, or di-PEG(5K)-AF13948, has increased stability, *i.e.,* an increased half-life, over the non-PEGylated form of the peptide. As such, Figure 1 indicates that the PEGylated peptides have good bioavailability and increased stability in human serum.

### Example 6

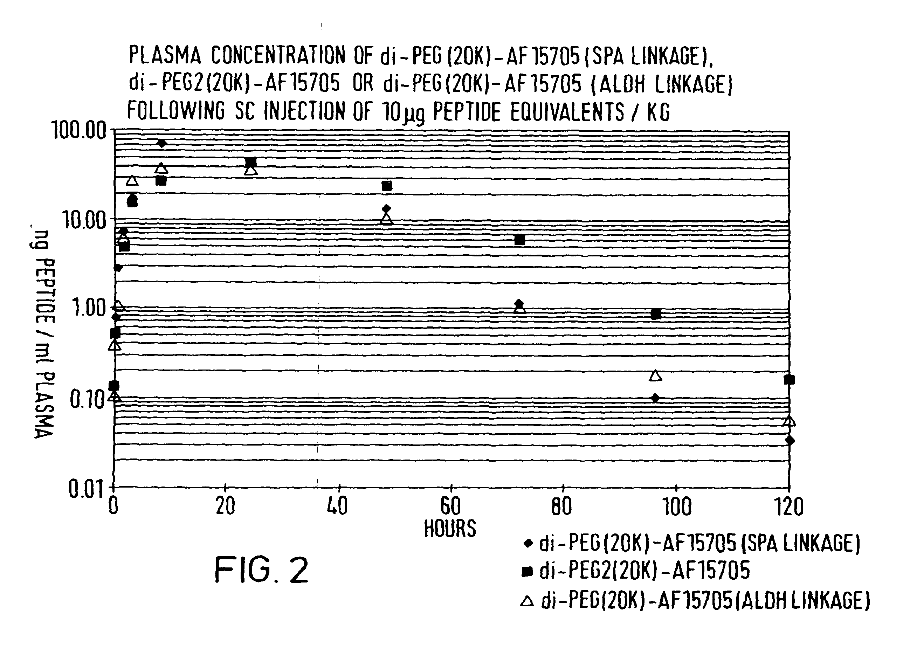

Using the assay described above, the pharmacokinetic profile of a peptide variously derivatized with PEG was determined. In this experiment, the peptide was derivatized with branched PEG2(20K), with an ester linked PEG (SPA) and with an aldehyde linked PEG (ALDH) as illustrated in Schemes 1-3. Figure 2 shows the plasma concentrations following SC injection of 10µg peptide equivalents/kg. From Figure 2, it is apparent that all three of the peptide compounds variously derivatized with PEG have favorable pharmacokinetic profiles.

### EXAMPLE 7

In this experiment, PEGylated peptides were evaluated for their effect on thrombocytopenia in a mouse model. In this assay, mice are made thrombocytopenic by treating the Balb/C mice with carboplatin (90 mg/kg intraperitoneally) on Day 0. GW350781 (1 mg/kg/day), or di-PEG(5K)-AF13948, and GW350805 (32.5 µg/kg/day), or di-PEG(20K)-AF13948, were given on Days 1-9 (s.c., qd). From Figures 3-4, it is apparent that the PEGylated peptides ameliorate carboplatin-induced thrombocytopenia on about Day 10. These results clearly indicate that the PEGylated peptides of the invention can ameliorate thrombocytopenia in a mouse model.

### EXAMPLE 8

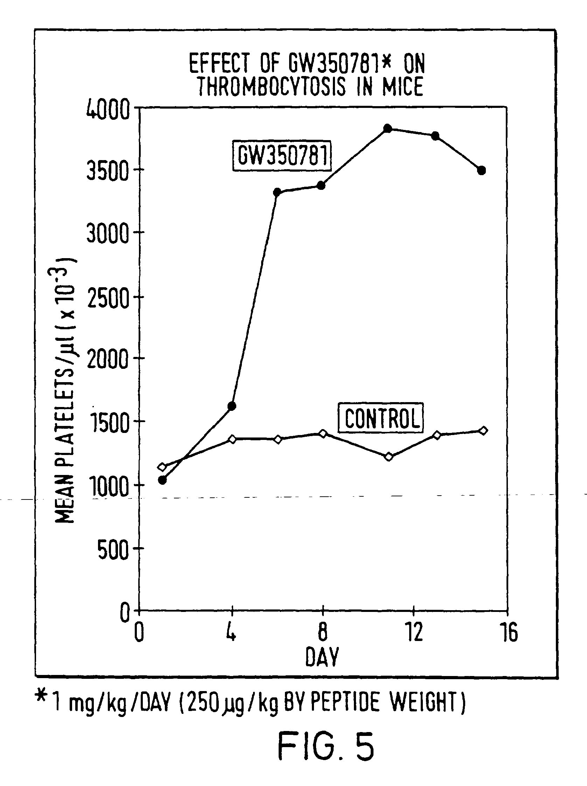

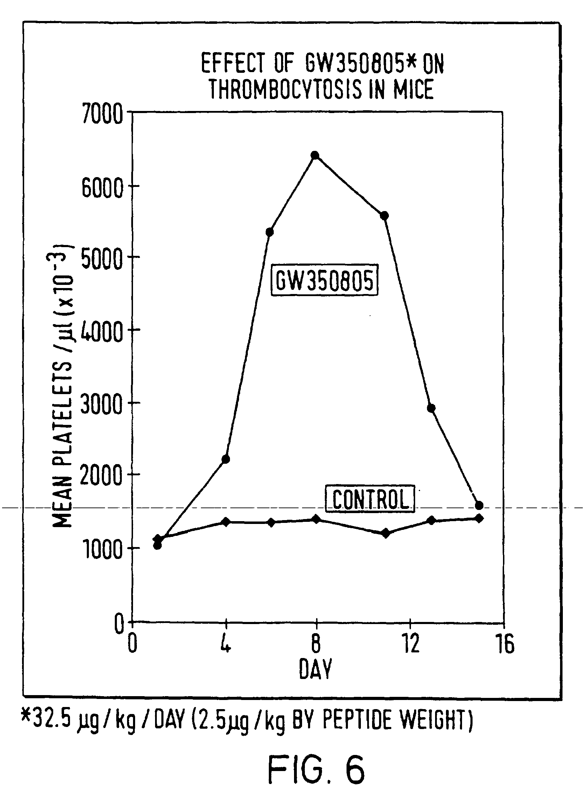

In this experiment, PEGylated peptides were evaluated for their effect on platelet levels in normal mice. In one experiment, GW350781 (1 mg/kg/day) and GW350805 (32.5 µg/kg/day) were given on Days 1-9 (s.c., qd). On days 1-15, blood was sampled at intervals by tail vein bleeds. From Figures 5-6, it is apparent that the PEGylated peptides have an effect on thrombocytosis, with the 20K-diPEGylated peptide GW50805 being about 100-fold more potent than the 5K-PEGylated peptide GW350781.

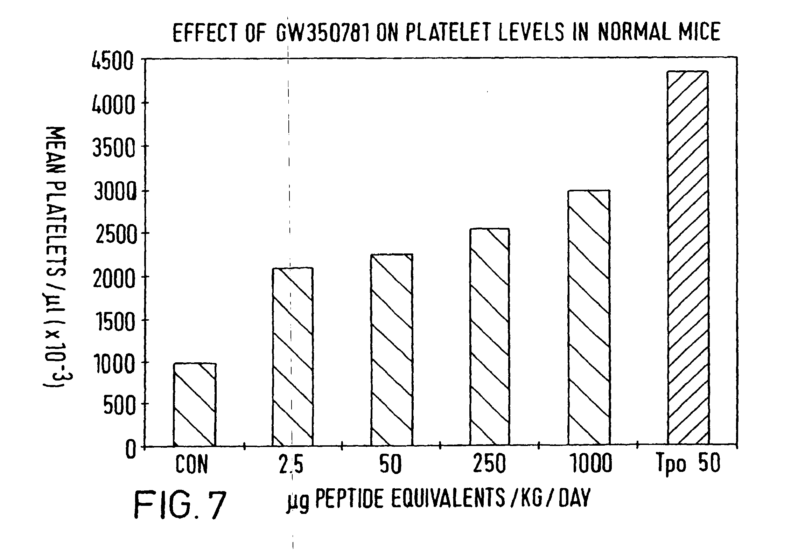

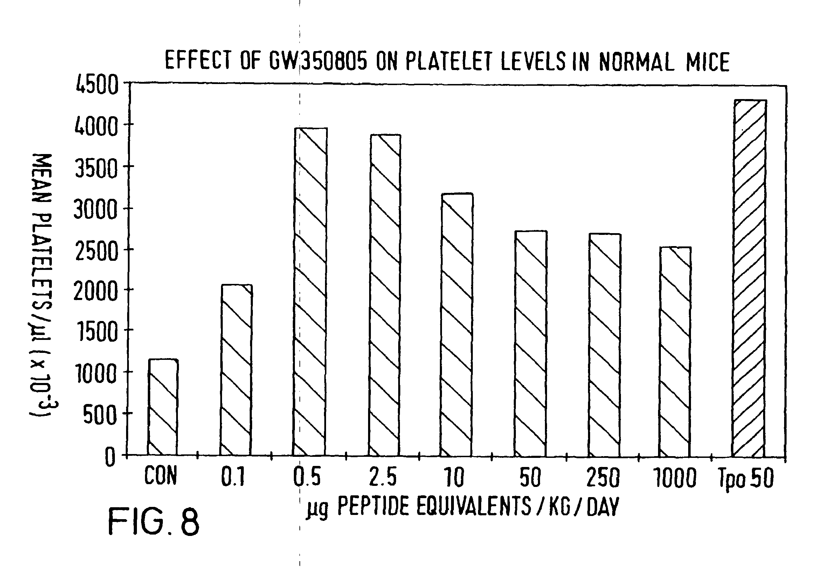

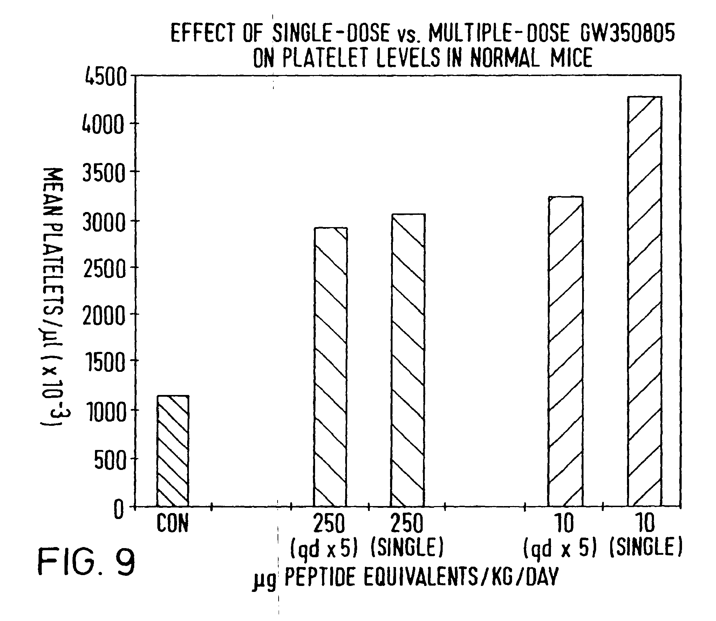

In another experiment, GW350781and GW305805 were given on Days 1-5 (s.c., qd). On day 6, the animals were sacrificed and peripheral blood platelet counts were obtained. Figures 7-9 set forth the effects of varying doses of the PEGylated peptides as well as the effects of single-dose versus multiple-dose. Such results clearly indicate that the PEGylated peptides of the invention can be used to increase platelets in a mouse model.

The disclosures in this application of all articles and references, including patent documents, are incorporated herein by reference in their entirety for all purposes.

## Claims

1. A compound that binds to the thrombopoietin receptor, wherein said compound comprises the following sequence:
X1 I E X2 P T L X3 X4 X5 L X6 X7 X8 X9 X10
where X1 is hydrogen or acyl;
X2 is G;
X3 is R;
X4 is Q;
X5 is W or a pharmaceutically acceptable derivative thereof;
X6 is A;
X7 is A;
X8 is R;
X9 is sarcosine (Sar); and
X10 is absent.

2. A compound that binds to the thrombopoietin receptor, wherein said compound comprises a protein dimer, and wherein each monomer of said protein dimer comprises the following sequence:
X1 I E X2 P T L X3 X4 X5 L X6 X7 X8 X9 X10
where X1 is hydrogen or acyl;
X2 is G;
X3 is R;
X4 is Q;
X5 is W or a pharmaceutically acceptable derivative thereof;
X6 is A;
X7 is A;
X8 is R;
X9 is sarcosine (Sar); and
X10 is absent.

3. The compound of claim 2, wherein the two monomers of said protein dimer are - - connected through a lysine linker.

4. The compound of any one of claims 1-3, wherein said compound is covalently attached to a hydrophilic polymer.

5. The compound of claim 4, wherein said hydrophilic polymer has an average molecular weight of between 500 and 40,000 daltons.

6. The compound of claim 5, wherein said hydrophilic polymer has an average molecular weight of between 5,000 and 20,000 daltons.

7. The compound of claim 6, wherein said hydrophilic polymer has an average molecular weight of 20,000 daltons.

8. The compound of claim 4, wherein said hydrophilic polymer is selected from the group consisting of polyethylene glycol, polypropylene glycol, polylactic acid and polyglycolic acid.

9. The compound of claim 8, wherein said hydrophilic polymer is polyethylene glycol.

10. The compound of claim 9, wherein said polyethylene glycol has an average molecular weight of between 500 and 40,000 daltons.

11. The compound of claim 10, wherein-said polyethylene glycol has an average molecular weight of between 5,000 and 20,000 daltons.

12. The compound of claim 11, wherein said polyethylene glycol has an average molecular weight of 20,000 daltons.

13. A pharmaceutical composition for thrombocytopenia comprising a compound of any one of claims 1-12 and a pharmaceutically acceptable carrier.

14. A method of activating a thrombopoietin receptor in a cell derived from a non-human, comprising contacting said cell with an effective amount of a compound of any one of claims 1-12.
